# EUROPEAN PATENT APPLICATION

(11) **EP 0 535 521 A2**
(43) Date of publication of application: **07.04.1993**
(21) Application number: 92116249.1
(22) Date of filing: 23.09.1992
(51) Int. Cl.: C07D 413/00, C07D 277/82, C07D 413/12, C07D 417/12, C07D 513/04, C07D 498/04, A61K 31/42, A61K 31/425, A61K 31/44

(54) **Condensed oxazole and thiazole derivatives as leukotriene biosynthesis inhibitors**

(30) Priority: 24.09.1991 US 764591; 04.09.1992 US 937315
(71) Applicant: BOEHRINGER INGELHEIM PHARMACEUTICALS INC., Ridgefield, Connecticut 06877-0368 (US)
(72) Inventor: Lazer, Edward S., Trumbull, Connecticut 06611 (US); Adams, Julian, Ridgefield, Connecticut 06877 (US); Miao, Clara K., Trumbull, Connecticut 06611 (US); Farina, Peter, North Salem, New York 10560 (US)
(74) Representative: Laudien, Dieter, Dr.

(57) **Abstract**

Substituted benzoxazoles, benzothiazoles, oxazolopyridines and thiazolopyridines of the general formula I 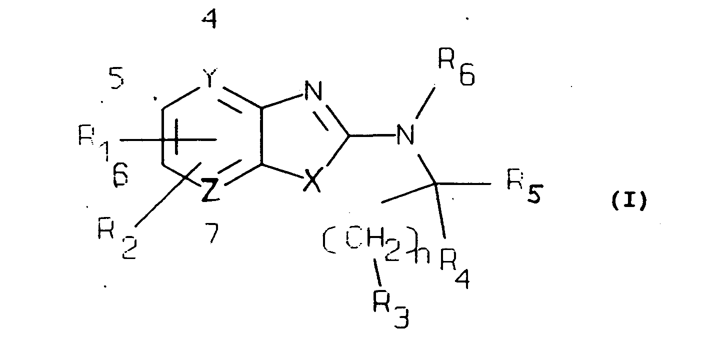
are described and disclosed, which compounds are potent inhibitors of leukotriene synthesis in warm-blooded animals.

## Description

This invention relates to leukotriene biosythesis inhibiters, to processes for their preparation, to compositions containing them and to their use.

Leukotrienes (LTs) are potent, pro-inflammatory substances that are produced in the metabolism of arachidonic acid. Research results lead to the conclusion that LTs play a role in various inflammatory diseases, such as asthma, allergic rhinitis, rheumatoid arthritis, inflammatory bowel disease and psoriasis. Accordingly, inhibition of the biosynthesis of LTs has potential utility in the treatment of diseases in which inflammation and arachidonic acid metabolism have been implicated.

The biosynthesis of leukotrienes and their pathophysiology have been well documented. Many investigators have been seeking to block the pathophysiological effects of leukotrienes either by blocking their biosynthesis or by blocking their activity at the receptor level. Two recent reviews (J.H. Musser and A.F. Kreft, J. Med. Chem. 1992, 35,2501 and A. Shaw and R.D. Krell, J. Med. Chem. 1991, 34, 1235) describe the status of research in these areas, including results of clinical trials. Results of clinical trials such as those cited in these articles support the concept that agents that block the biosynthesis or activity of leukotrienes will be useful in asthma and other inflammatory diseases mentioned above.

According to one aspect of this invention, we provide various substituted benzoxazoles, benzothiazoles, oxazolopyridines and thiazolopyridines which have the ability to inhibit leukotriene biosynthesis. Such compounds have the general formula (I): 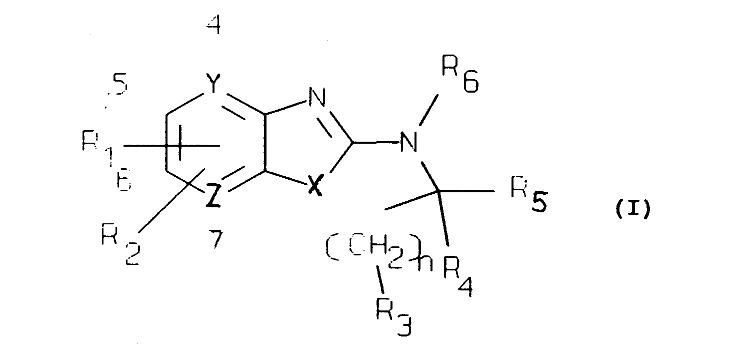
wherein
X is 0 or S;
Y is C or N;
Z is C or N;
R₁ and R₂ are each, independently of one another, hydrogen; C1-C6 alkyl; halo; CF₃; nitrile; C₁-C₆ alkoxy; - CO₂R₇ wherein R₇ is hydrogen or Ci-C₆ alkyl; -C(O)NR₈R₉ wherein R₈ and R₉ are independently hydrogen, C₁-C₃ alkyl, methoxy or together with the N atom form a morpholine, pyrrolidine or piperidine ring; -N0₂; -NR₁₀R₁₁ wherein R₁₀ and R₁₁ are independently hydrogen or C₁-C₆ alkyl;-C(O)R₁₂ wherein R₁₂ is C₁-C₆ alkyl; -SO₂R₁₂; -NHC(O)R₁₂;-NHSO₂R₁₂; or -SO₂NR₁₃R₁₄ wherein R₁₃ and R₁₄ are independently hydrogen or C₁-C₆ alkyl;
R₃ is methyl, cyclohexyl or an unsubstituted or substituted phenyl ring wherein the substituents are selected from halo, CF₃, C₁-C₄ alkyl and C₁-C₄ alkoxy; N0₂; SO₂R₁₂, -NHC(O)R12, -NHSO₂R₁₂ or -SO₂NR₁₃R₁₄ wherein R₁₂, R₁₃ and R₁₄ are as defined above; a 1-piperidinyl ring, a 2-, 3- or 4- pyridine ring, a morpholine ring, a thiomorpholine ring, a pyrrolidine ring, an imidazole ring optionally substituted at the nitrogen with C₁-C₄ alkyl, or a 2-thiazole ring or a 2-methyl-4-thiazole ring; a dialkylamine (C₁-C₄) or an alkyl ether (C₁-C₄);
R₄ is an ester of structure -CO₂R₁₆ wherein R₁₆ is G₁-C₄ alkyl; an amide of structure -C(O)NR₁₇R₁₈ wherein R₁₇ and R₁ are independently hydrogen, C₁-C₃ alkyl, methoxy or together with the N atom form a morpholine ring, piperidine or pyrrolidine ring; an unsubstituted or substituted phenyl ring wherein the substituents are selected from halo, C₁-C₄ alkyl or C₁-C₄ alkoxy; a 3-methyl-1,2,4-oxadiazol-5-yl group; a 2-or 3-thienyl group; a 2-, 3-, or 4-pyridyl group; a 4-pyrazolyl group; a 2-imidazole group optionally substituted at the N atom with a methyl group; a 2-thiazole group optionally substituted at the 4-position with a methyl; a ketone of structure C(O)R₁ wherein R₁ is C₁-C₃ alkyl, phenyl or 1-methylimidazol-2-yl; an ether CH₂OR₂₀ where R₂₀ is C₁-C₃ alkyl; a thioether - CH₂SR₂₀; a sulfone - CH₂S0₂CH₃; an amine - CH₂N(R₂ₒ)₂; an amine derivative - CH₂NHC(O)R₂₁, where R₂₁ is CH₃ or NHCH₃; or -CH₂NHS0₂Me₂; or a carbamate - CH₂0C(O)NHMe;
R₅ and R₆ are independently of each other hydrogen or methyl; and
n is an integer 0, 1 or 2;
with the proviso that Y and Z are not both N; and with the further provisos that the following combination of substituents do not occur simultaneously:
   (i) Y and Z are both carbon; R₁ or R₂ are hydrogen, halo, C₁-C₄ alkyl, C₁-C₄ alkoxy, CN, N0₂ or CF₃; R₃ is an unsubstituted phenyl; and R₄ is -C(O)OR₁₆ wherein R₁₆ is hydrogen, alkyl, alkenyl or alkynyl; or R₄ is C(O)N(R_{18'})(R_{19'}) wherein R_{18'} and R_{19'} are hydrogen, C₁-C₆alkyl, phenyl or alkoxy or together with the N atom form a pyrrolidine, piperidine or morpholine ring; or R₄ is - CN or -C(S)NH₂;
   or (ii) Y and Z are both carbon; R₄ is C(O)OCH₃; R₁ and R₂ are both hydrogen, and R₃ is 4-hydroxyphenyl, unsubstituted phenyl or a 4-imidazole group.

Such compounds may be provided in racemic form, or the pure or substantially pure enantiomers may be isolated. The preferred compounds are those wherein the R₁ substituent is in the 5-position and is an C₁-C₃ alkyl group or halogen, the R₃ substituent is cyclohexyl, the R₆ substituent is hydrogen and n is 1.

In J. Pharm. Sci (57, p. 1695) by S. Advani and J. Sam (1968) four compounds are disclosed having a basic structure similar to that of Formula (I). The Advani publication, a publication on potential antineoplastic agents, discloses synthesis of these four compounds, but no biological activity is provided. In the Advani publication, Y and Z are both carbon, R₄ is -C0₂C₂Hs, R₁ and R₂ are both hydrogen and R₃ is 4-C₆H₄OH, -C₆H₅, 4-imidazolyl or -CH2SCH3.

In DE 3419994 there are described compounds of general formula (I) wherein both Y and Z are C, X is O or S, R₁ and R₂ are hydrogen, halo, C₁-C₄ alkyl, C₁-C₄ alkoxy, CN, N0₂ or CF₃, and R₄ is -C(O)OR_{16'}-(wherein R_{16'} is hydrogen, alkyl, alkenyl or alkynyl), or - C(O)N(R₁₈)(R₁₉)(wherein R₁₈ and R₁₉ are hydrogen, C₁-C₆ alkyl, phenyl or alkoxy or together with N form a pyrrolidine, piperidine or morpholine ring), or -CN or - C(S)NH₂. The group referred to as "Y" in DE 3419994 consists of R₃ and the carbon chain, i.e. (CH₂)_{n'} shown in Formula (I) above. The groups at "Y" disclosed in the German publication are straight-chain or branched alkyl (Cₗ-C₈), with one to three carbons between N and R_{4'} or the group designated "Z" is methylthioalkyl (one to three carbons in the alkyl) or phenylalkyl. Specifically not disclosed at R₃ is a cyclohexyl group or substituted phenyl. Also, not disclosed at R₄ are ketones or phenyl and heteroaromatic or heterocyclic rings. Finally, also not disclosed in the German publication is Y or Z being nitrogen. At R₆ the German publication discloses hydrogen and C₁-C₄ alkyl. Specifically not disclosed are the preferred compounds of the present invention. Compounds disclosed in the German publication are said to be useful for protecting crops against certain classes or types of herbicides.

The compounds of the present invention may be prepared by a variety of processes and such processes comprise further aspects of the invention. These are essentially similar to those known in the art and published in the literature. For example, compounds may be prepared by reaction of an appropriately substituted 2-chlorobenzoxazole, 2-chlorobenzothiazole, 2-chlorooxazolopyridine or 2-chlorothiazolopyridine with an amine, an amino acid or an amino acid ester. Such synthesis scheme is outlined herein below as Scheme A.

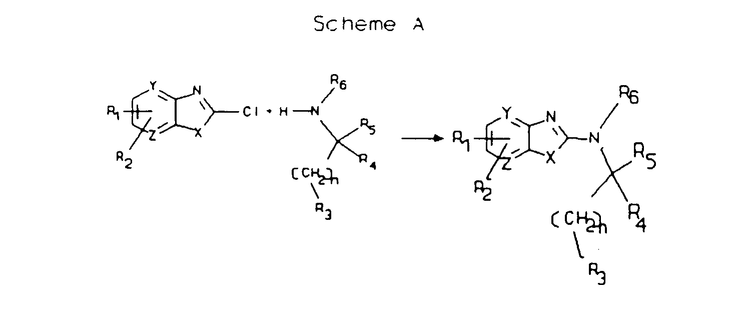

The reaction of Scheme A may occur in an inert solvent, such as methylene chloride, toluene or DMSO, with a basic catalyst, such as triethylamine or NaOH. The optimum choice of both solvent and catalyst will depend on the nature of the reactants, as a person skilled in the art would recognize.

Alternatively, modification of a procedure known in the literature for preparation of 2-aminoben- zothiazoles may be successfully employed for synthesis of compounds of general formula I. Such synthesis scheme is outlined hereinbelow as Scheme B.

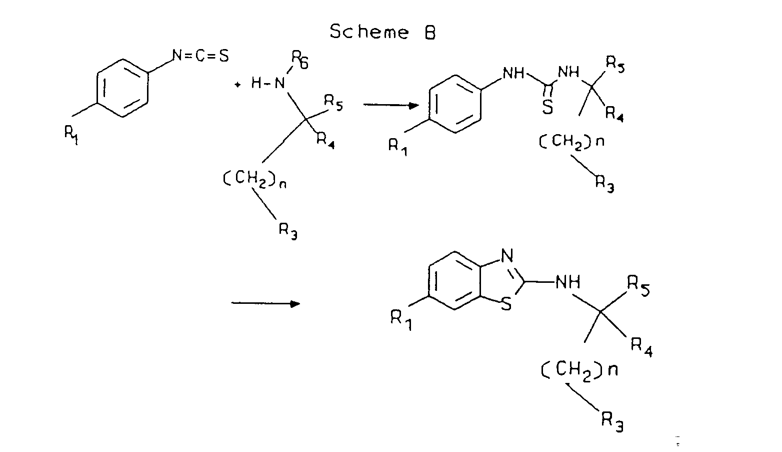

The procedure of Scheme B involves reaction of an appropriately substituted isothiocyanate with an amine or an amino acid ester in a suitable inert solvent, such as ether, followed by cyclization of the intermediate thiourea formed e.g. with sulfuryl chloride or bromine in another inert solvent, again such as ether or perhaps chlorobenzene.

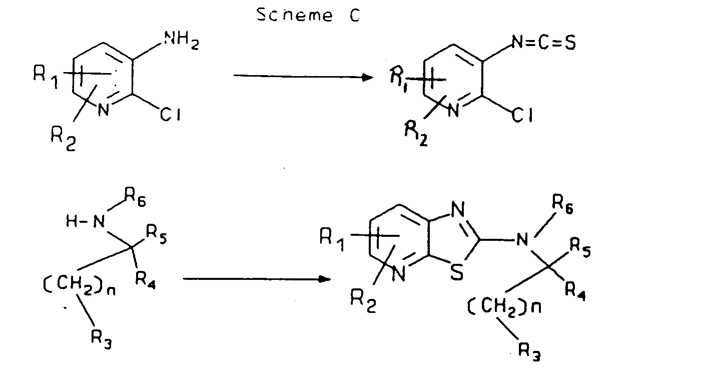

The synthesis of Scheme C can be employed for those compounds of general formula I wherein X is S and Z is N. A haloaminopyridine is converted to an isothiocyanate, for example by reaction with thiophosgene, in the presence of a base, such as sodium carbonate, in an inert solvent. Treatment of the isothiocyanate with an amine in an inert solvent yields a thiazolopyridine. With certain additional substituents on the 2-chloro-3-aminopyridine ring, an intermediate thiourea is isolated upon reaction with the isothiocyanate. In that case, cyclization to the thiazolopyridine product may be accomplished by heating in an inert solvent with either acid or base catalysis, for example in ethanolic HCI or DMF with K₂C0₃.

Isomeric thiazolopyridines may be prepared by cyclization of a 3-halo-2-thiourea substituted pyridine. Such a synthetic scheme is outlined hereinbelow as Scheme D.

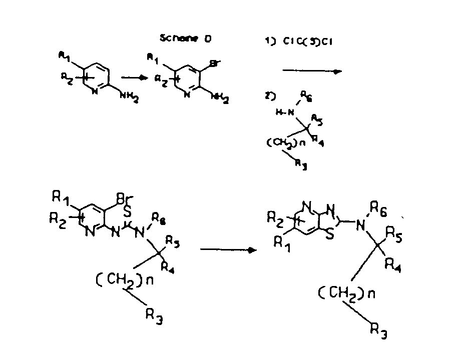

In Scheme D, the 3-halo-2-thiourea pyridine is heated in an inert solvent with base catalysis, for example K₂C0₃ in DMF. The intermediate 2-amino-3-halopyridine may be prepared, for example, by bromination of an optionally substituted 2-aminopyridine. The isothiocyanate may be prepared as described in Scheme C.

Compounds of general formula (I) wherein R₄ is an acid or an ester may be modified to yield compounds of general formula (I) wherein R₄ is an amide, a methyloxadiazole, a ketone, an ether, or thioether. One such scheme for modification is shown hereinbelow as Scheme E.

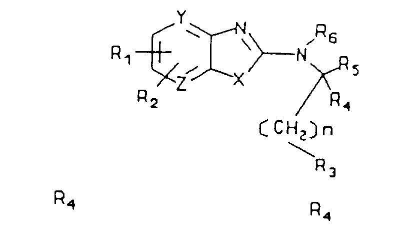

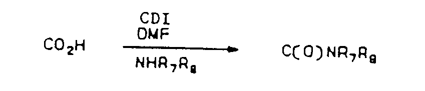

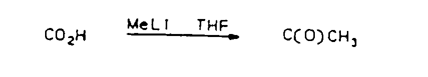

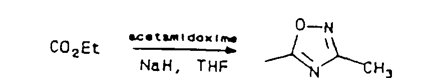

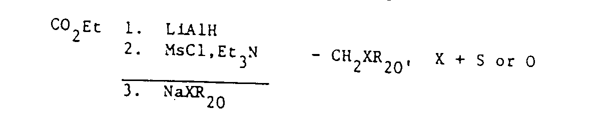

All the general methods exemplified in Scheme E are well known to one skilled in the art, and are also published in the chemical literature.

Concerning the stereochemistry of compounds produced via the processes and schemes outlined hereinabove, if the starting amines used above are enantiomerically pure, then a single enantiomer of the end product, having either R or S configuration at the asymmetric carbon, will be recovered. By the same token, if the starting amine is racemic, that is, a mixture of R and S, then a racemic end product will be recovered. Racemic compounds may be separated into the individual enantiomers by methods known to one skilled in the art, for example, by resolution of a diastereomeric salt, chromatography on a chiral column, etc. In the text of this specification the designation for enantiomers of amino acids D and L, or racemic DL, will be used.

The following examples are illustrative of the present invention. As would be obvious to one skilled in the art, other compounds, such as where R₄ is pyrrolidine-amide, can be readily synthesized using the methods and procedures outline above.

### Preparation

### DL-N-(Benzothiazol-2-yl)phenylalanine hydrochloride

11.8 g phenylalanine (71.4 mmol) was added in portions to a suspension of 5.7 g powdered NaOH (143 mmol) in 50 ml DMSO, and stirred under nitrogen. 2-Chlorobenzothiazole (11 g, 65 mmol) was added over fifteen minutes at room temperature. The reaction was heated on an oil bath at about 95 ° C for 4 hours. The reaction mixture was then cooled, poured into 200 ml ice and water, and the pH of the resulting mixture was adjusted to about 1-2 by addition of 10N HCI.

More ice was added, and the mixture was then stirred and filtered. The white solid was dissolved in alkaline solution, stirred with celite, filtered, acidified with 2N HCI and filtered. The resulting solid was rinsed with water, then EtOH, and dried. The product (6.47 g, 19.3 mmol, 30%) melted at 250-251 ° C.

### Example 1

### DL-N-(Benzothiazol-2-yl)phenylalanine ethyl ester

### Compound No. 4, Table 1

Thionyl chloride (3.82 g, 32.1 mmol) was added dropwise to the product from the above preparation (3.2 g, 10.7 mmol) suspended in 200 ml EtOH. The reaction was heated at reflux for four hours. The reaction mixture was then concentrated, the residue dissolved in EtOAc (75ml) and extracted with saturated Na₂C0₃ solution (2 X 50 ml), saturated NaCl solution (50 ml) dried (Na₂S0₄) and concentrated. The product was recrystallized from EtOH giving 2.25 g (6.9 mmol, 64%) mp 137-139 °C.

### Example 2

### DL-N-(6-lsopropylbenzothiazol-2-yl)-4-chlorophenylalanine ethyl ester

### Compound No. 7, Table 1

DL-4-Chlorophenylalanine ethyl ester hydrochloride(5g, 18.9 mmol) was converted to the free base using triethylamine. A solution of the free base in 75 ml ether was added to a solution of 4-isopropyl- phenylisothiocyanate in 150 ml ether, and cooled on an ice-salt bath. The temperature was maintained at about 0 ° C during addition. The reaction was stirred for four and one-half hours, at which time the reaction temperature was 12 ° C. The reaction mixture was filtered, the filtrate concentrated, and the resulting foamy residue triturated with petroleum ether while cooling on ice. This resulted in 6.1 g (15.1 mmol, 80%) N-(4-isopropylphenyl)-N'-[2-(4-chlorophenyl)-1-(ethoxycarbonyl)ethyl]thiourea, mp 73-75 ° C.

The intermediate product (6 g, 14.8 mmol) was dissolved in 25 ml chlorobenzene and cooled on an ice bath to 0 ° C. Sulfuryl chloride (2.76 g, 20.4 mmol) in 5 ml chlorobenzene was added dropwise. After five and one-half hours, the reaction mixture was concentrated, the residue dissolved in EtOAc (150 ml), washed with saturated Na₂C0₃ solution, then saturated NaCl solution, dried (Na₂S0₄) and concentrated. The product crystallized from EtOH, giving 4.07g(10.1 mmol, 68%), mp 105-107 °C.

### Example 3

### 2-(2-Cyclohexyl-1-phenyl)ethylaminobenzoxazole

### Compound No. 51, Table 3

A mixture of 1.12 g 2-chlorobenzoxazole (7.3 mmol), 1.48 g 2-cyclohexyl-1-phenylethylamine (7.3 mmol) and 0.88 g triethylamine (8.8 mmol) in 305 ml CH₂CI₂, was refluxed for 31 hours. The reaction mixture was diluted with 50 ml CH₂ C1₂, extracted with water (1 X 50 ml), 1 N HCI (1 X 50 ml), saturated NaCl (1 X 50 ml), dried (Na₂S0₄) and concentrated. The resulting solid was recrystallized from EtOH giving 1.4 g product (4.4 mmol, 60%) mp 129-131 ° C.

### Example 4

### L-2-[2-Cyclohexyl-1-(3-methyl-1,2,4-oxadiazol-5-yl)ethyl]amino-5-methylbenzoxazole

### Compound No. 125, Table 6

0.47 g 60% NaH in mineral oil dispersion (0.28 g NaH, 11.8 mmol), 0.39 g acetamidoxime (5.3 mmol), 1.48 g N-(5-methylbenzoxazol-2-yl)cyclohexylalanine methyl ester (4.7 mmol), and several molecular sieves were combined in 20 ml THF and refluxed for two hours under nitrogen. The mixture was poured into water and extracted with EtOAc. The EtOAc was dried (Na₂S0₄) and concentrated. The product was purified by flash chromatography on silica gel (99 CH₂CI₂ : 1 MeOH). After triturating with petroleum ether the product was obtained as a white solid, 70 mg, mp 118-119 ° C.

### Example 5

### L-N-(5-Methylbenzoxazol-2-yl)cyclohexylalanine-N'-methylamide

### Compound No. 44, Table 2

A solution of 1.08 g L-N-(5-methylbenzoxazol-2-yl)cyclohexylalanine (3.6 mmol) in 15 ml CH₂CI₂ was cooled on an ice bath. Carbonyldiimidazole (0.88g, 5.4 mmol) was added in portions. After one hour methylamine gas was bubbled into the reaction mixture for about forty-five minutes. The reaction was diluted with CH₂CI₂, washed with water, saturated NaCl solution, dried (Na₂SO₄) and concentrated. The product was purified by flash chromatography on silica gel, eluting with 99 CH₂CI₂:1 MeOH, followed by recrystallization from isopropanol giving 0.2 g product, m.p. 202-204 ° C.

### Example 6

### 3-[(6-lsopropylbenzothiazol-2-yl)amino]-4-phenylbutan-2-one

### Compound No. 128, Table 7

A solution of 2g N-(6-isopropylbenzothiazol-2-yl)phenylalanine (5.9 mmol) in 60 ml THF was cooled on an ice-salt bath to -5 ° C, under nitrogen. A solution of 1.4 N MeLi in ether (26 ml, 36.4 mmol) was added via syringe over about one minute. After two hours 10ml chlorotrimethylsilane (78 mmol) was added rapidly and the reaction warmed up to room temperature. The reaction was quenched with 1 N HCI and the product extracted into ether, dried (Na₂SO₄) and concentrated. The product was purified by flash chromatography through silica gel, eluting with CH₂CI₂. Recrystallization from EtOH gave 0.75g product (2.2 mmol, 38%), mp 107-110°C.

### Example 7

### 2-[(2-Cyclohexyl-1-phenylethyl)amino]thiazolol[5,4-b] pyridine

### Compound No. 172, Table 11

A mixture of 1.28 g (10 mmol) 3-amino-2-chloropyridine, 2.1 g (20 mmol) sodium carbonate and 1.38 g (12 mmol) thiophosgene in 50 ml CH₂CI₂ was stirred overnight at room temperature. The reaction mixture was poured into water, and extracted with CH₂CI₂. The combined organic extracts were washed with saturated NaCl solution, dried (Na₂S0₄) and concentrated to give an oil. The product was purified by chromatography on silica gel, eluting with petroleum ether, giving 1.6 g thioisocyanate (9.4 mmol, 94%).

The thioisocyanate (0.516 g, 3.02 mmol) was added to a mixture of 0.66 g 2-cyclohexyl-1-phenylethylamine hydrochloride (2.75 mmol), and 0.278 g triethylamine (2.75 mmol) in 25 ml THF. The reaction was heated at reflux for two hours, poured into water, and extracted with ether. The organic extracts were washed with saturated NaCl solution, dried (MgS0₄) and concentrated. Recrystallization of the residue from CH₂CI₂ - petroleum ether gave 0.625 g product (1.84 mmol, 67%) mp 146-148°C.

### Example 8

### 2-{[2-Cyclohexyl-1-(2-pyridyl)ethyl]amino}-6-methylthazolo-[4,5-b]pyridine

### Compound No. 184, Table 11

Bromine (3.19 g) was added dropwise at 0°C to a solution of 2-amino-5-picoline in 75 ml CH₂ C1₂. After about two hours at room temperature, the reaction was extracted with saturated sodium carbonate solution, then sodium thiosulfate solution. The combined aqueous extracts were washed with CH₂CI₂, and the combined organic extracts washed with saturated NaCI, dried (Na₂S0₄) and concentrated giving 3.59 g crude material. The product was purified by flash chromatography on silica gel, eluting with petroleum ether with increasing amounts of CH₂ Cl₂ (0-40%), giving 3.05 g 2-amino-3-bromo-5-picoline, m.p. 68-70 C.

To the above product (2.84 g, 15 mmol) in 50 ml CH₂CI₂ with 3.18 g (30 mmol) sodium carbonate, was added 2.07 g (18 mmol) thiophosgene. After stirring overnight at room temperature, the reaction was extracted with water, the aqueous phase back extracted with CH₂CI₂ and combined organic extracts washed with brine, dried (MgS0₄), and concentrated giving the isothiocyanate as an oily material that crystallized on standing (3.9 g) IR 2050 cm-¹.

To a solution of 1.0 g (4.3 mmol) of the isothiocyanate derivative and 1.04 g (4.3 mmol) of 2-cyclohexyl-1-phenylethylamine in 50 ml dry THF was added 438 mg (4.3 mmol) of Et₃N. The resulting mixture was refluxed for two hours. The triethylamine hydrochloride was filtered off and the filtrate concentrated to give the thiourea (1.6 g) which crystallized on standing.

A mixture of 540 mg (1.15 mmol) of the thiourea and 317 mg (2.3 mmol) K₂C0₃ in 10 ml DMF was refluxed overnight. The reaction mixture was then poured into water and extracted with ether(3X) and CH₂CI₂ (1X). The organic extracts were washed with brine, dried (Na₂SO₄) and concentrated giving 450 mg product. Recrystallization from CH₂CI₂/ether/petroleum ether gave 210 mg product, m.p. 213-214° C.

According to a further aspect of the present invention, compounds of formula (I) as defined above appear useful in therapy.

According to another aspect of the present invention, we provide pharmaceutical compositions comprising as active ingredient at least one compound of formula (I) 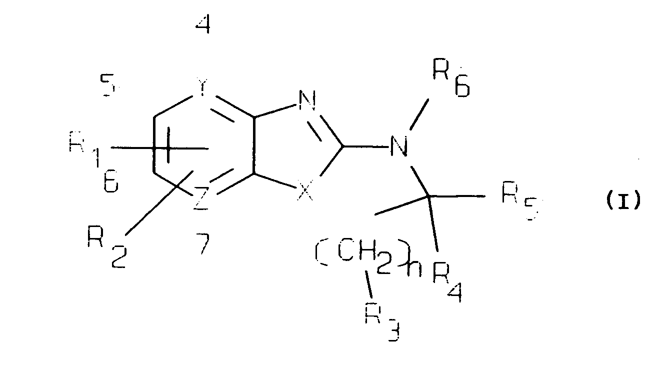
wherein
X is O or S;
Y is C or N;
Z is C or N;
R₁ and R₂ are each, independently of one another, hydrogen; C₁-C₆ alkyl; halo; CF₃; nitrile; C₁-C₆ alkoxy; - CO₂R₇ wherein R₇ is hydrogen or Ci-C₆ alkyl; -C(O)NR₈R₉ wherein R₈ and R₉ are independently hydrogen, C₁-C₃ alkyl, methoxy or together with the N atom form a morpholine, pyrrolidine or piperidine ring; -N0₂; -NR₁₀R₁₁ wherein R₁₀ and R₁₁ are independently hydrogen or C₁-C₆ alkyl;-C(O)R₁₂ wherein R₁₂ is C₁-C₆ alkyl; -SO₂R₁₂; -NHC(O)R₁₂;-NHSO₂R₁₂; or -SO₂NR₁₃R₁₄ wherein R₁₃ and R₁₄ are independently hydrogen or C₁-C₆ alkyl;
R₃ is methyl, cyclohexyl or an unsubstituted or substituted phenyl ring wherein the substituents are selected from halo, CF₃, C₁-C₄ alkyl and C₁-C₄ alkoxy; N0₂; SO₂R₁₂, -NHC(O)R₁₂, -NHSO₂R₁₂ or -SO₂NR₁₃R₁₄ wherein R₁₂, R₁₃and R₁₄ are as defined above; a 1-piperidinyl ring, a 2-, 3- or 4- pyridine ring, a morpholine ring, a thiomorpholine ring, a pyrrolidine ring, an imidazole ring optionally substituted at the nitrogen with C₁-C₄ alkyl, or a 2-thiazole ring or a 2-methyl-4-thiazole ring; a dialkylamine (C1 -C4) or an alkyl ether (C₁-C₄);
R₄ is an ester of structure -C0₂R,6 wherein R₁₆ is G₁-C₄ alkyl; an amide of structure -C(O)NR₁₇R₁₈ wherein R₁₇ and R₁ are independently hydrogen, C₁-C₃ alkyl, methoxy or together with the N atom form a morpholine ring, piperidine or pyrrolidine ring; an unsubstituted or substituted phenyl ring wherein the substituents are selected from halo, C₁-C₄ alkyl or C₁-C₄ alkoxy; a 3-methyl-1,2,4-oxadiazol-5-yl group; a 2-or 3-thienyl group; a 2-, 3-, or 4-pyridyl group; a 4-pyrazolyl group; a 2-imidazole group optionally substituted at the N atom with a methyl group; a 2-thiazole group optionally substituted at the 4-position with a methyl; a ketone of structure C(O)R₁ wherein R₁ is C₁-C₃ alkyl, phenyl or 1-methylimidazol-2-yl; an ether CH₂0R₂o where R₂₀ is G₁-C₃ alkyl; a thioether - CH₂SR₂₀; a sulfone - CH₂S0₂CH₃; an amine - CH₂N(R₂ₒ)₂; an amine derivative - CH₂NHC(O)R₂,, where R₂₁ is CH₃ or NHCH₃; or -CH₂NHS0₂Me₂; or a carbamate - CH₂0C(O)NHMe;
R₅ and R₆ are independently of each other hydrogen or methyl; and
n is an integer 0, 1 or 2;
with the proviso that Y and Z are not both N;
in racemic form, or the pure or substantially pure enantiomer thereof, or a physiologically acceptable acid addition salt thereof in association with one or more pharmaceutically acceptable carriers, diluents or excipients.

The compounds of this invention are potent inhibitors of leukotriene synthesis in warm-blooded animals. The compounds according to the present invention may be administered to warm-blooded animals topically, perorally, parenterally, rectally or by the respiratory route as active ingredients in conventional pharmaceutical compositions, that is, comprising a pharmaceutical carrier or excipient and an effective amount of the active ingredient.

In another aspect, the present invention provides pharmaceutical compositions comprising a compound of formula I or a physiologically acceptable salt thereof, together with at least one pharmaceutical carrier or excipient.

Suitable vehicles or carriers for the above noted formulations are described in standard pharmaceutical texts, e.g. in "Remington's Pharmaceutical Sciences", 17th ed, Mack Publishing Company, Easton, Penn., 1985.

The dosage of the active compounds will vary with the form of administration and the particular active agent chosen. Furthermore, it will vary with the particular host under treatment. Generally, treatment is initiated with small increments until the optimum effect under the circumstances is reached.

With reference to systemic administration, the compound of formula I is administered at a dosage of 10 mcg to 1000 mcg per kilogram of body weight per day, although the aforementioned variations will occur. However, a dosage level that is in the range of from about 50 mcg to 500 mcg per kilogram of body weight per day is most desirably employed in order to achieve effective results.

According to a yet further aspect of the present invention, we provide a method of preventing or treating inflammatory conditions or diseases or conditions in which arachidonic acid metabolism has been implicated in a warm blooded animal which comprises administering to the animal an effective amount of a compound of formula (I) as defined above in respect of compounds of formula (I) for use in the pharmaceutical compositions of this invention. Preferably, the method relates to the inhibition of the biosynthesis of leukotrienes.

According to another aspect of the present invention, we provide the use of compounds of formula (I) as defined above in respect of compounds of formula (I) for use in the pharmaceutical compositions of this invention, or physiologically acceptable salts thereof, for the preparation of medicaments for use in the prevention or treatment of inflammatory conditions and conditions or diseases in which arachadonic acid metabolism has been implicated. Such medicaments are also for use in the inhibition of the biosynthesis of leukotrienes.

### Inhibition of LTB₄, biosynthesis in human polymorphonuclear leukocytes (PMNs)

The inhibition of leukotriene biosynthesis is measured by determining whether and to what extent test compounds can inhibit L TB4 production from endogenous arachidonic acid in human peripheral blood leukocytes.

To 48-well tissue culture plates was added a solution of the test compound followed by addition of human polymorphonuclear leukocytes isolated from peripheral blood at a density of 1.5X10⁶ cells/well. Culture plates were preincubated for fifteen minutes with shaking at 28 °C. Cells were stimulated with calcium ionophore A23187 at a final concentration of 2.5 µM for an additional ten minutes. The reaction was terminated by the addition of an EGTA solution (10 mM final concentration) followed by centrifugation at 1500 rpm at 10°C. Supernatants were stored at -70°C. LTB₄ levels were determined by RIA using a commercially available kit. Nonlinear regression analysis was used to calculate IC50 values.

The following tables show % inhibition of LTB₄ biosynthesis by compounds of the invention at test concentrations indicated, with the determined IC₅₀ shown in µM.

Also, in all the following Tables, R₂ is a hydrogen atom unless a double entry is entered for R₁ whereupon R₂ is the second entry.

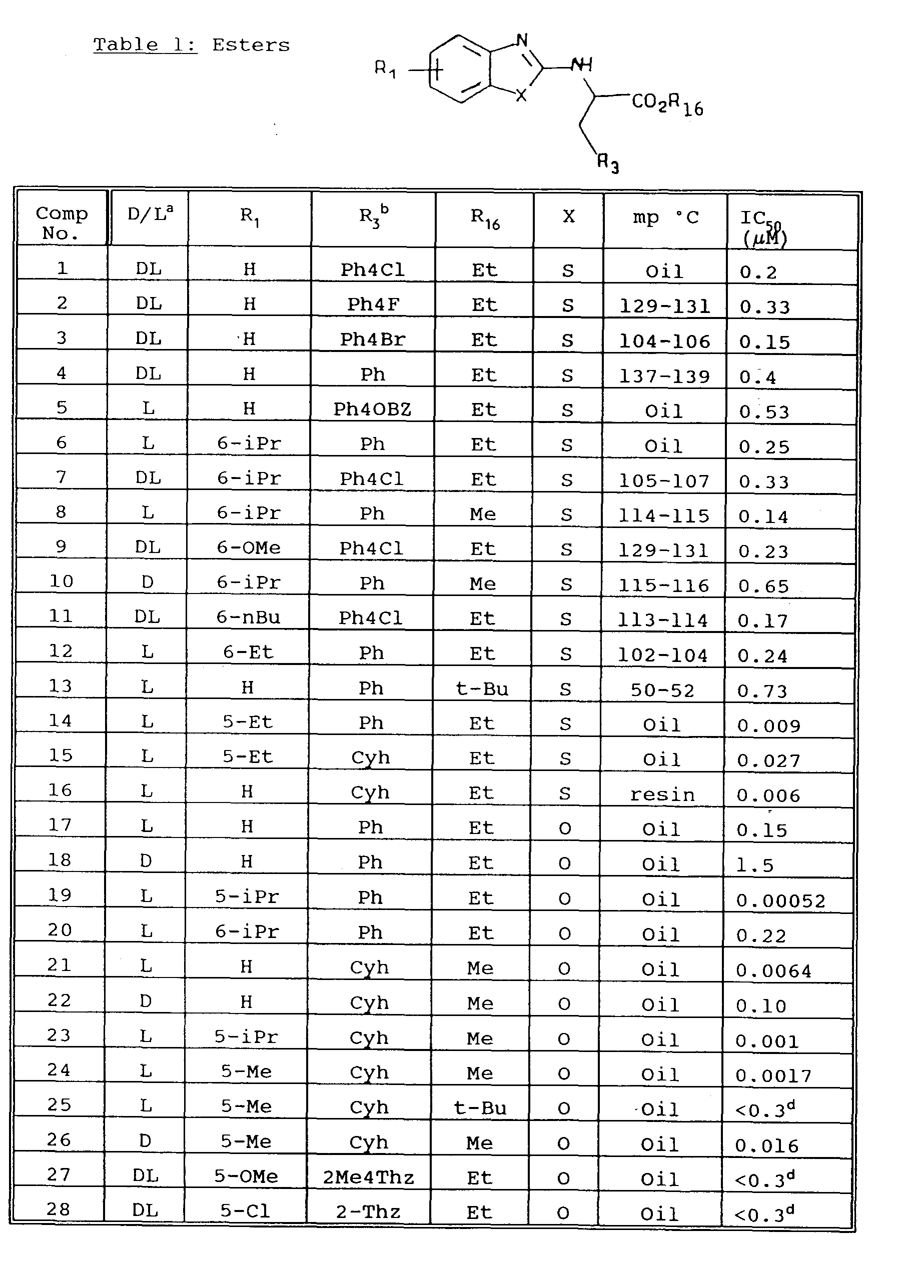

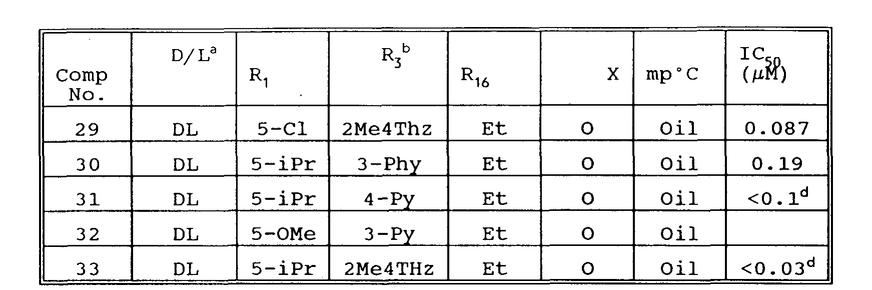

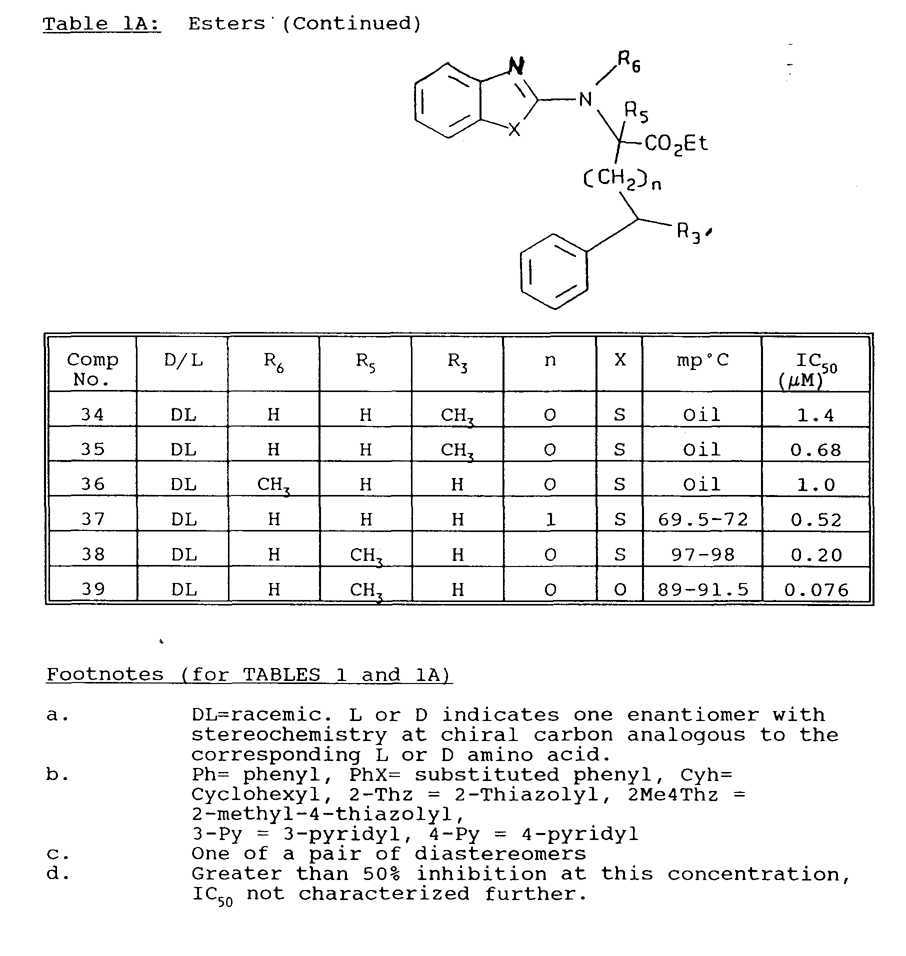

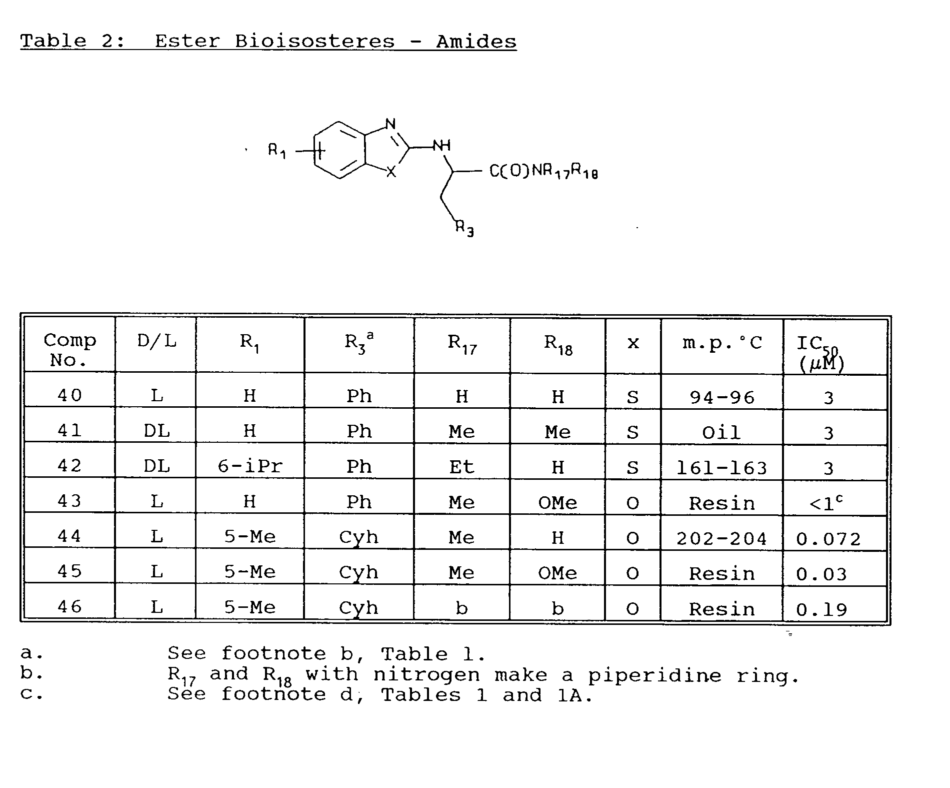

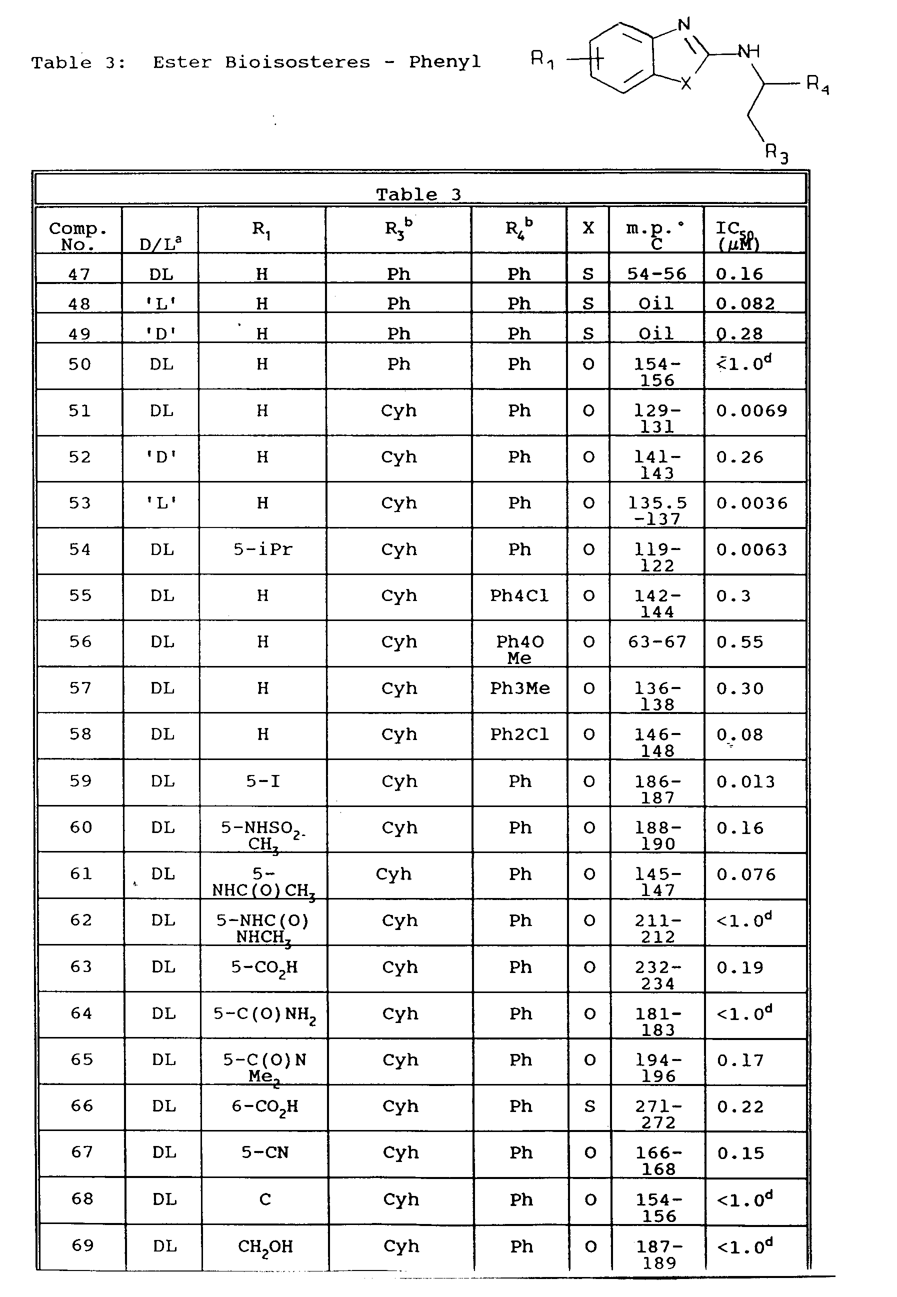

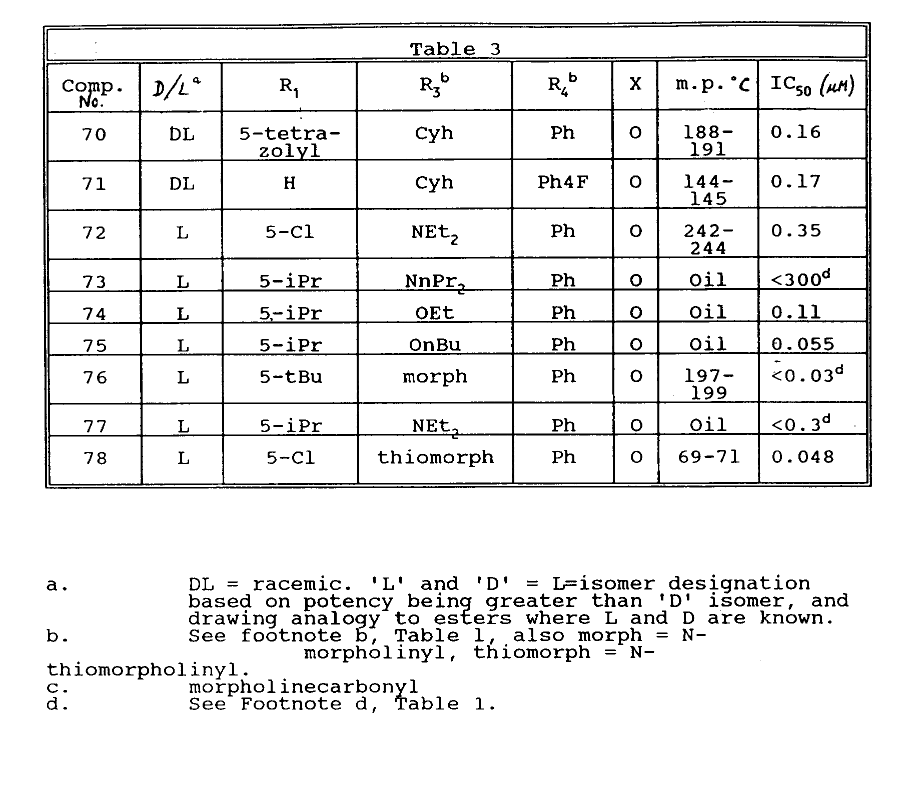

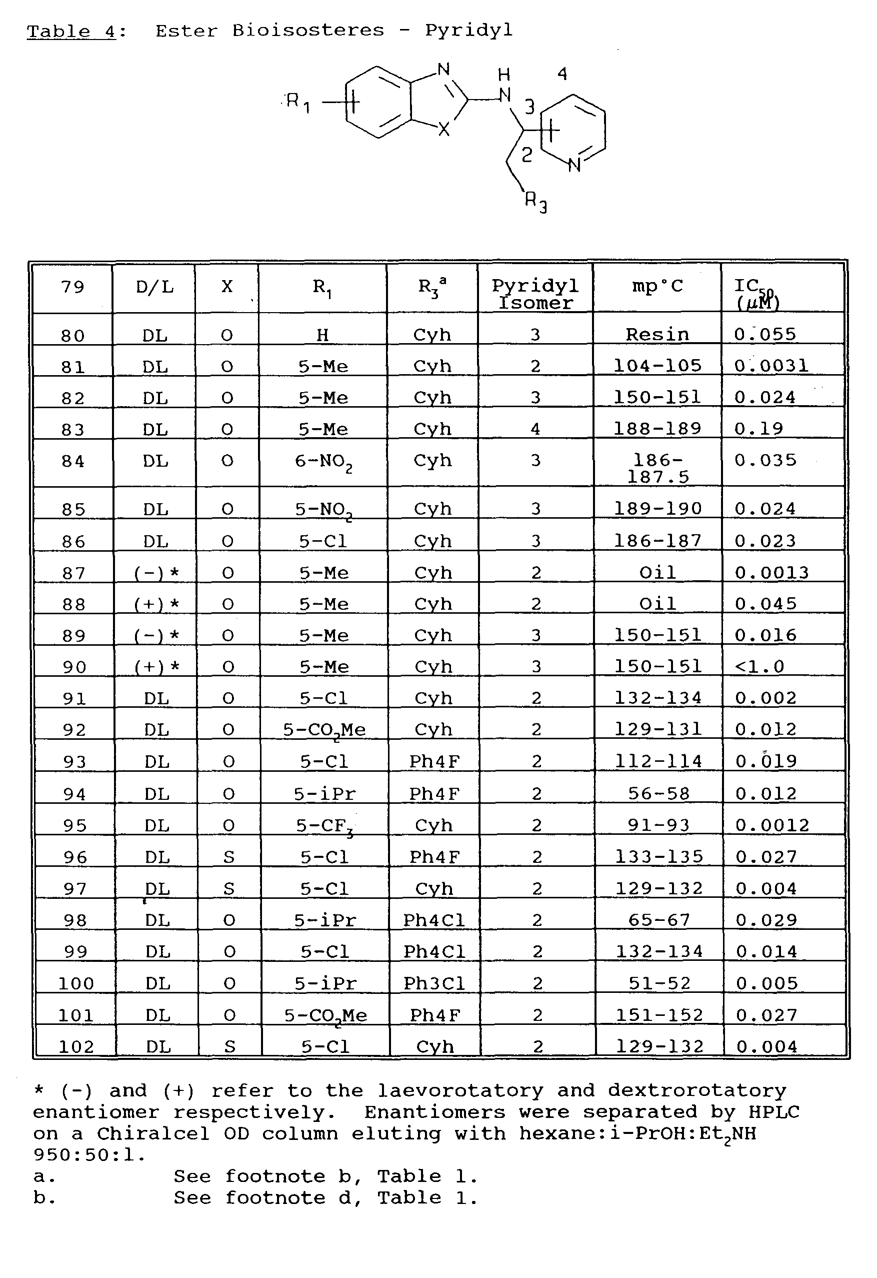

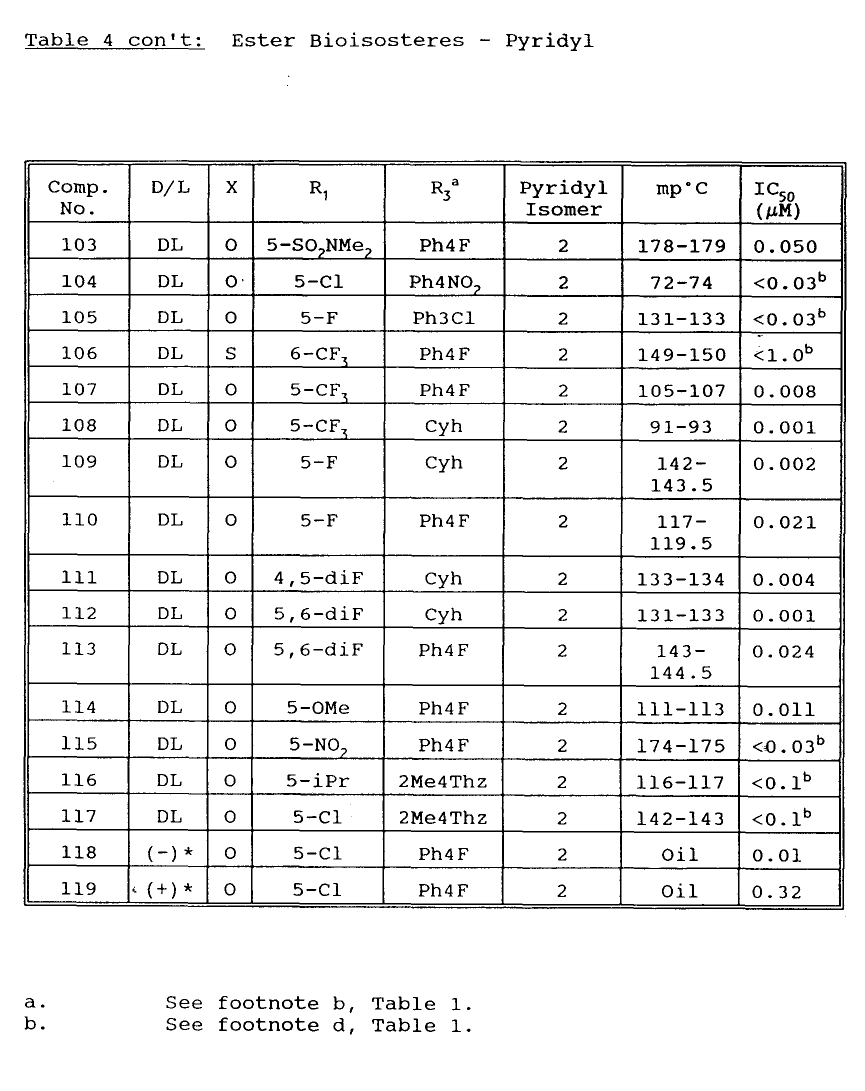

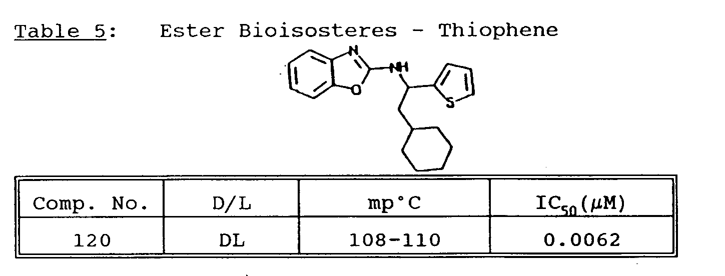

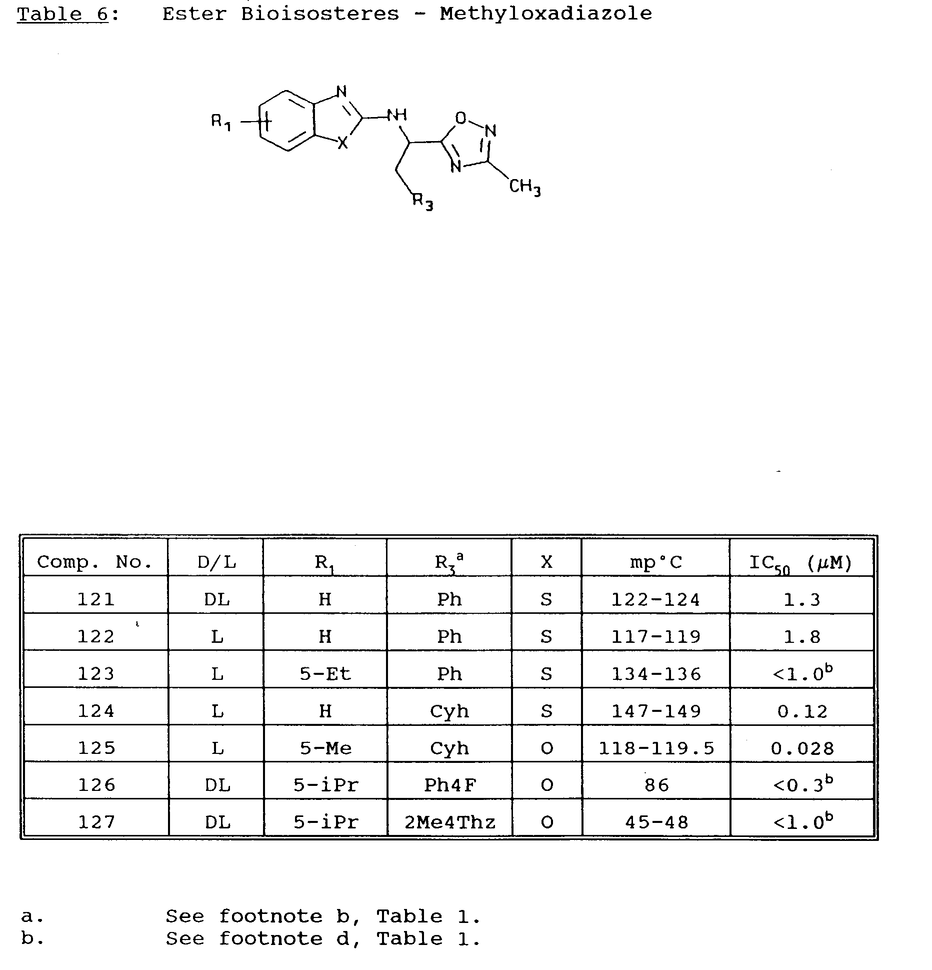

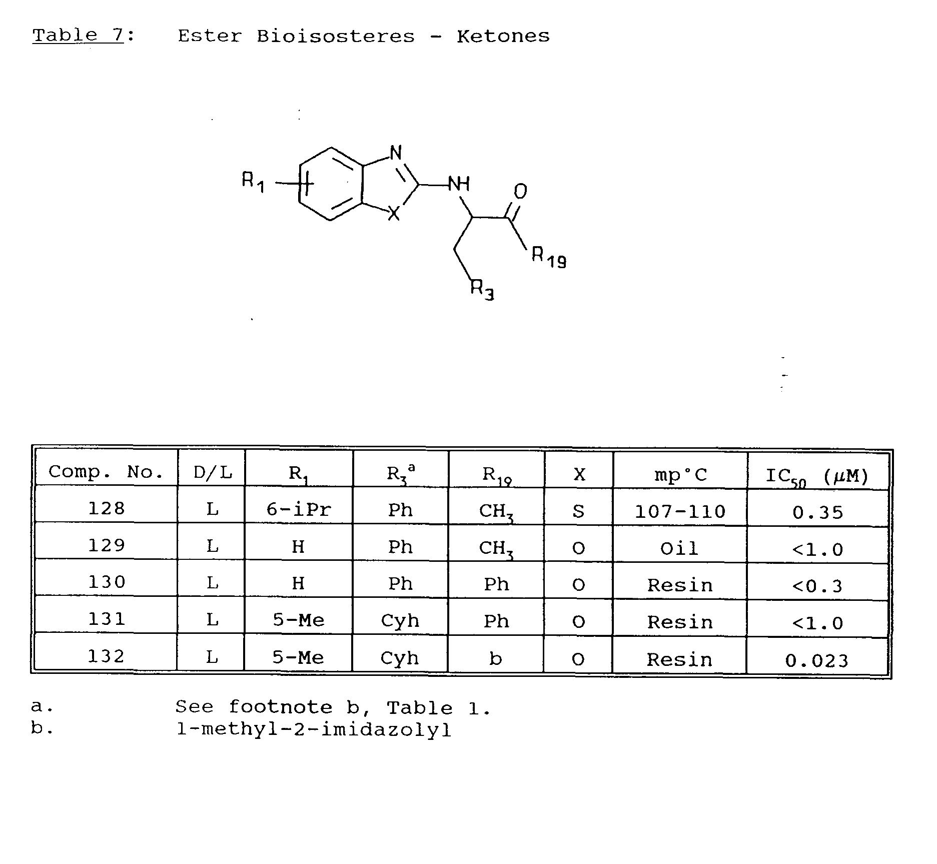

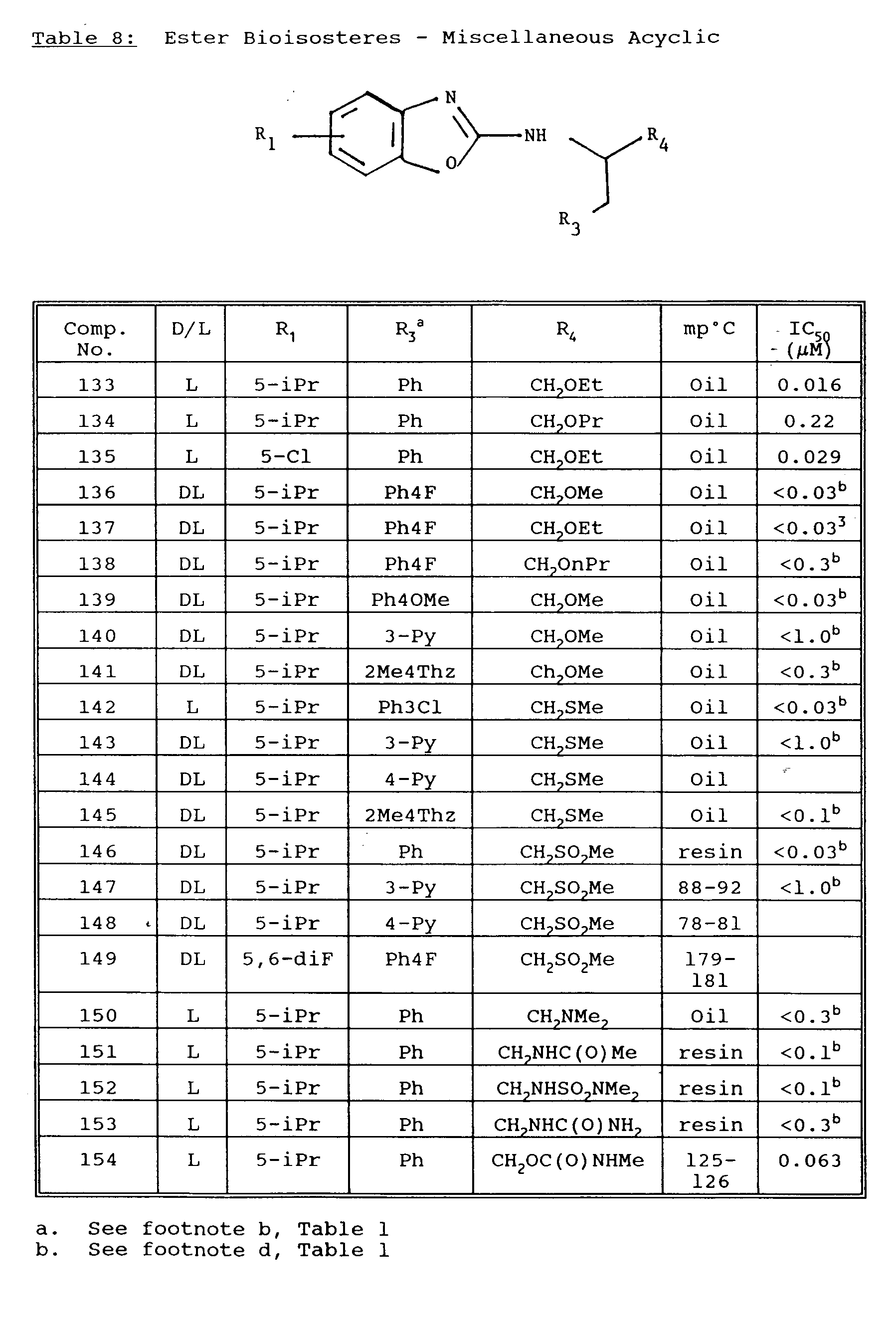

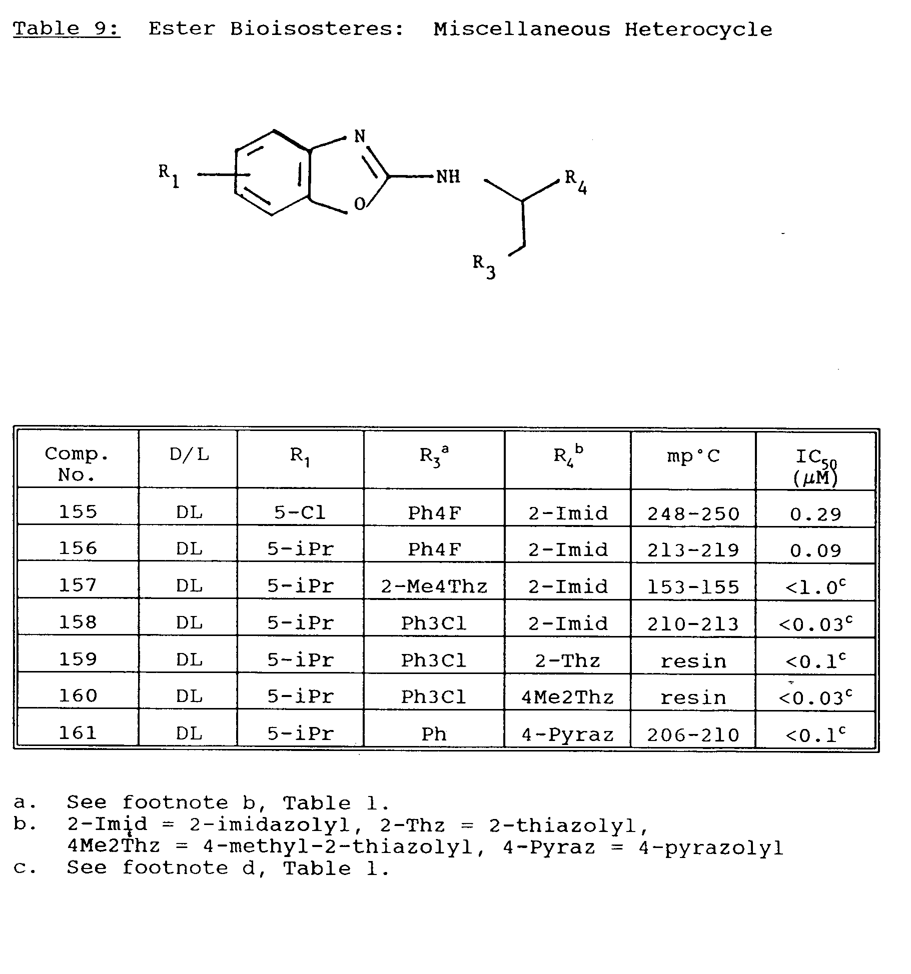

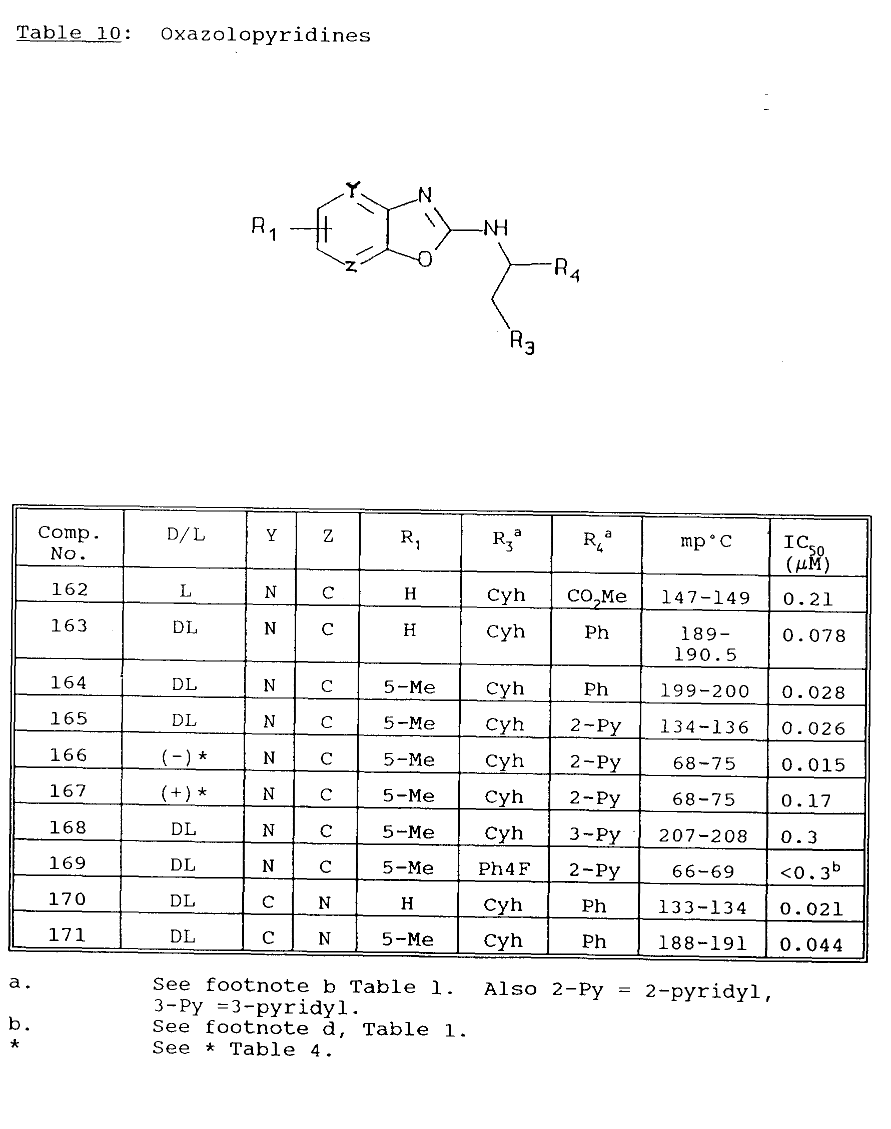

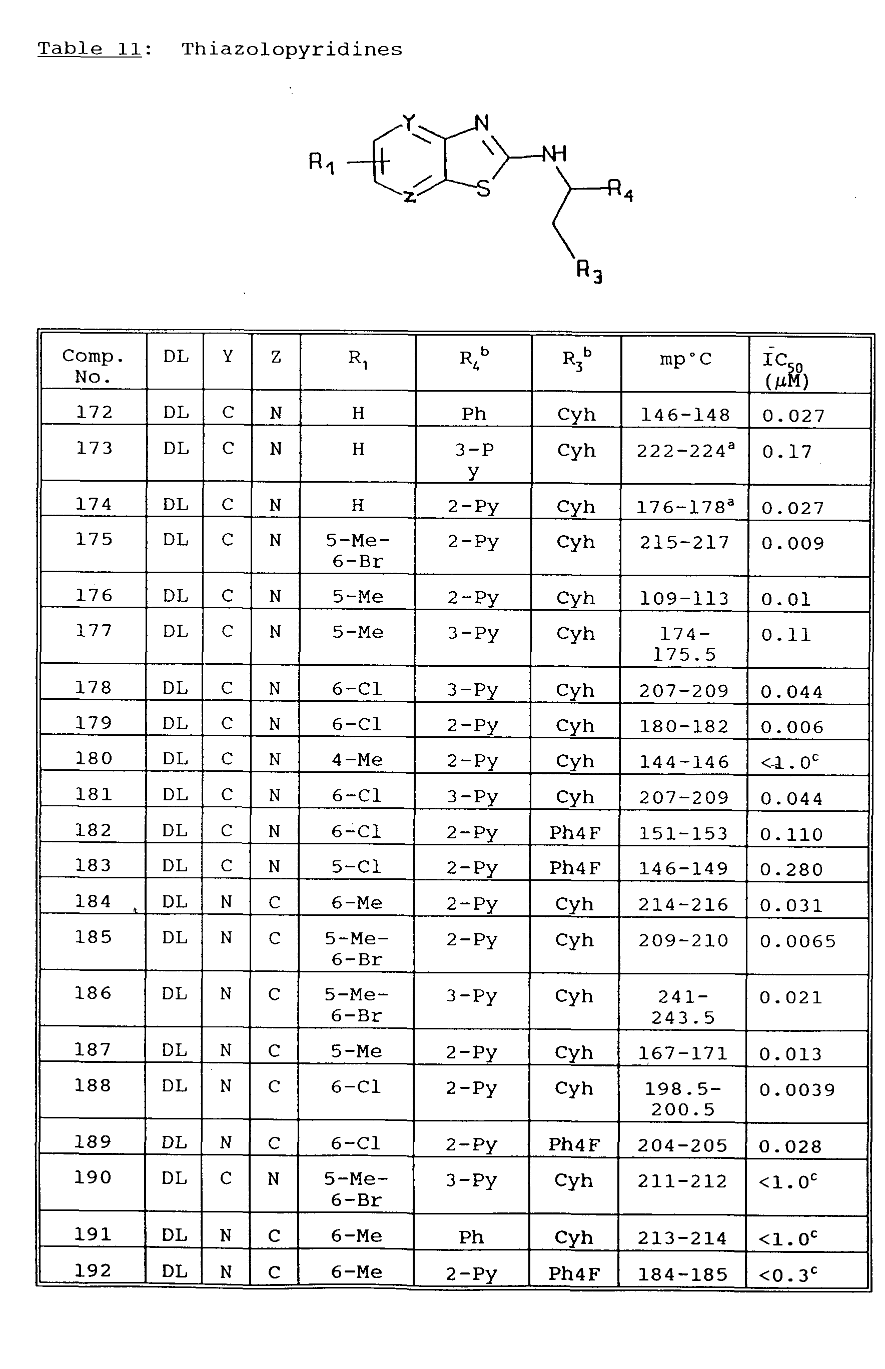

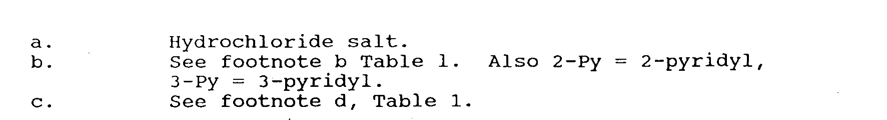

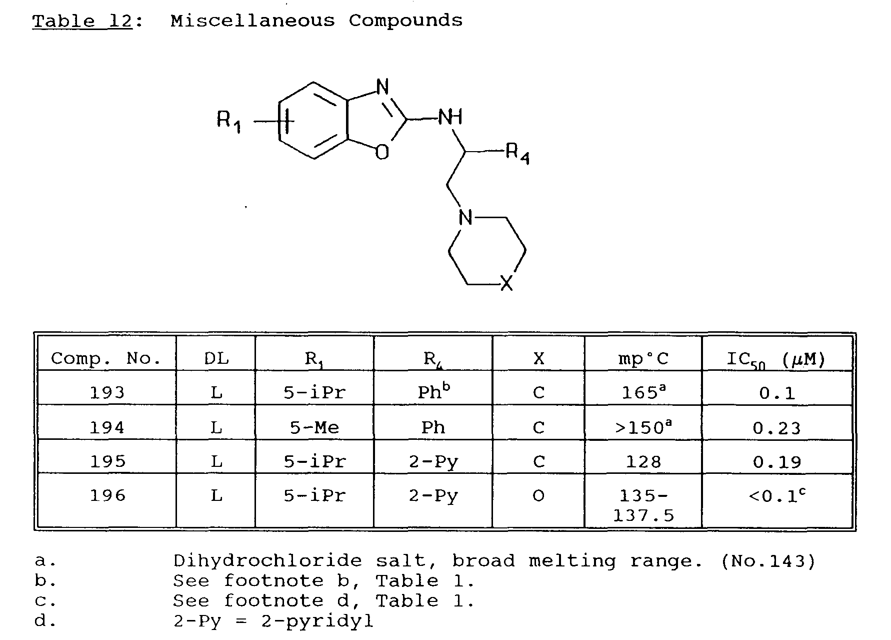

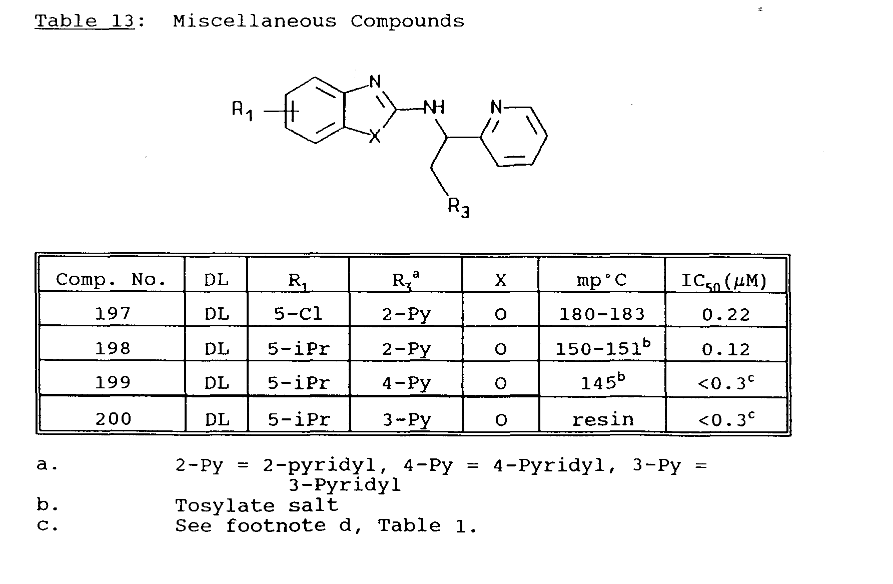

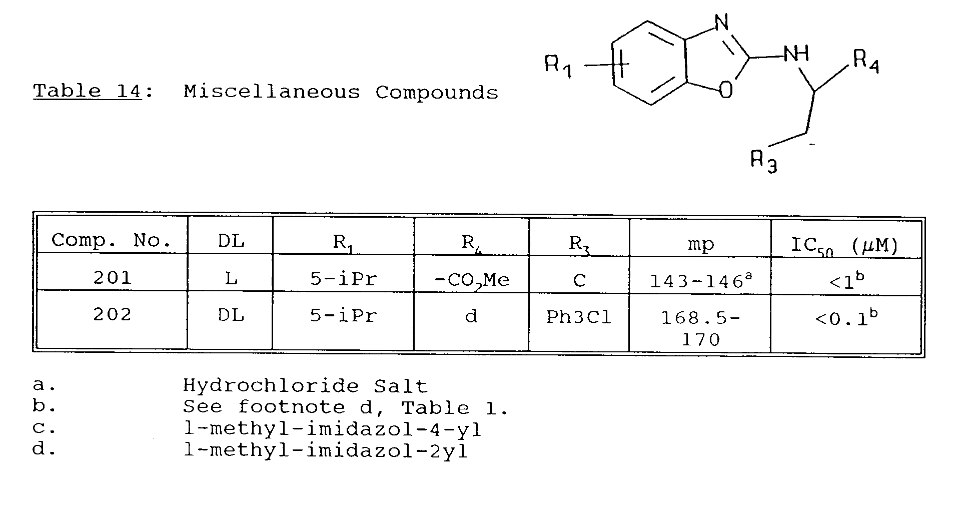

### Antigen-induced bronchoconstriction in guinea pigs.

This model measures the ability of a compound to block the leukotriene component of antigen-induced bronchoconstriction. Male Hartley guinea pigs are actively sensitized to ovalbumin, pretreated with mepyramine and indomethacin (to block the histamine and cyclooxygenase metabolite components respectively), and test compound (by aerosol administration). The guinea pigs are challenged with antigen (inhaled ovalbumin). Pulmonary function is measured by oscillatory mechanics as described by W.W. Wolyniec et al. (Agents and Actions 1991, 34, 1/2, 73). Results are expressed as percent inhibition of bronchoconstriction (resistance) in the test compound treated guinea pigs compared to placebo treated controls.

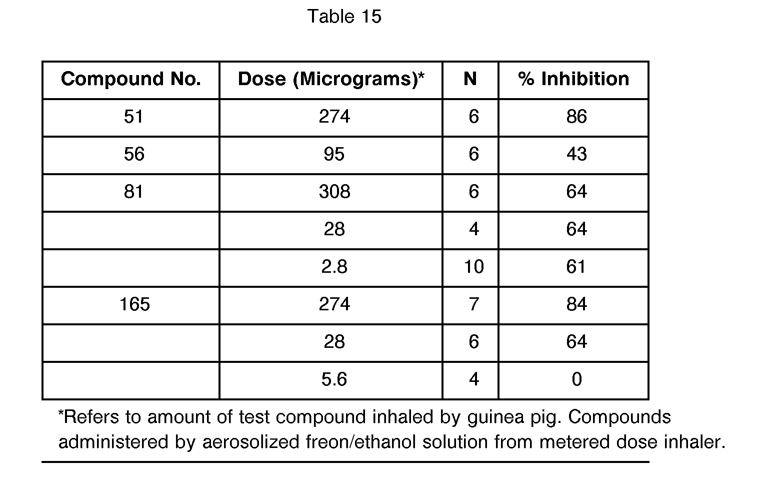

### Antigen-induced mediator release in primates.

This model measures the ability of a compound to inhibit the formation of leukotriene C₄ (LTC₄) in the lungs of allergic cynomolgus monkeys following antigen challenge. Animals are anesthetized, intubated, and challenged with an Ascaris suum extract, given by aerosol. A bronchoalveolar lavage is performed 20 minutes later and LTC₄ is quantitated by radioimmunoassay. Test compounds are adminstered by aerosol 10 minutes prior to antigen challenge and their effect is expressed as percent inhibition of LTC₄ production compared to untreated controls.

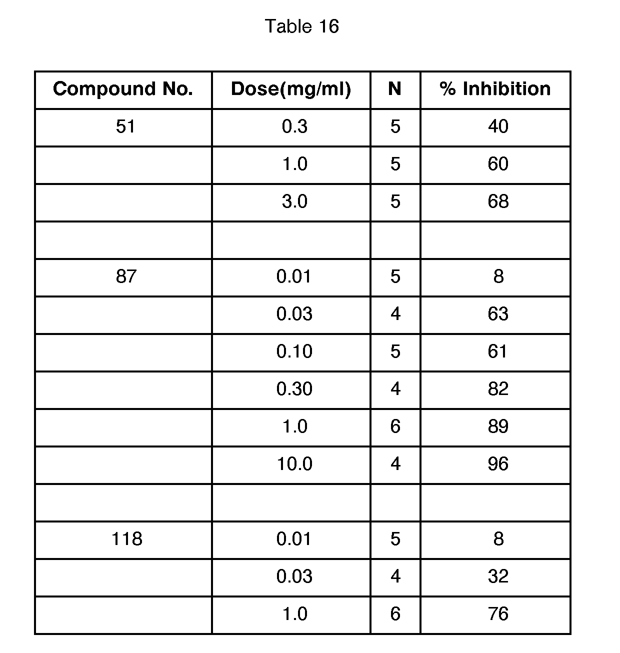

## Claims

1. Compounds of formula (I) 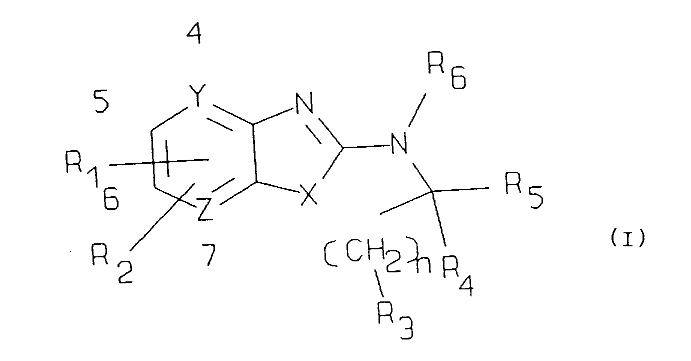
wherein
X is O or S;
Y is C or N;
Z is C or N;
R₁ and R₂ are each, independently of one another, hydrogen; C₁-C₆ alkyl; halo; CF₃; nitrile; C₁-C₆ alkoxy; -CO₂R₇ wherein R₇ is hydrogen or C₁-C₆ alkyl; -C(O)NR₈R₉ wherein R₈ and R₉ are independently hydrogen, C₁-C₃ alkyl, methoxy or together with the N atom form a morpholine, pyrrolidine or piperidine ring; -N0₂; -NR₁₀R₁₁ wherein R₁ and R₁₁ are independently hydrogen or C₁-C₆ alkyl;-C(O)-R₁ wherein R_{1 2} is C₁-C₆ alkyl; - SO₂R₁₂; -NHC(O)R_{1 2};-NHSO₂ R₁₂; or -S0₂ NR_{1 3} R_{1 4} wherein R_{1 3} and
R₁ are independently hydrogen or C₁-C₆ alkyl;
R₃ is methyl, cyclohexyl or an unsubstituted or substituted phenyl ring wherein the substituents are selected from halo, CF₃, C₁-C₄ alkyl and G₁-C₄ alkoxy; N0₂; SO₂R₁₂, -NHC(O)R₁₂, - NHSO₂R₁₂ or -SO₂NR₁₃R₁₄ wherein R₁₂, R₁₃ and R₁₄ are as defined above; a 1-piperidinyl ring, a 2-, 3- or 4-pyridine ring, a morpholine ring, a thiomorpholine ring, a pyrrolidine ring, an imidazole ring optionally substituted at the nitrogen with C₁-C₄ alkyl, or a 2-thiazole ring or a 2-methyl-4-thiazole ring; a dialkylamine (C₁-C₄) or an alkyl ether (C₁-C₄);
R₄ is an ester of structure -CO₂R₁₆ wherein R₁₆ is C₁-C₄ alkyl; an amide of structure -C(O)NR₁₇R₁₈ wherein R₁₇ and R₁₈ are independently hydrogen, C₁-C₃ alkyl, methoxy or together with the N atom form a morpholine ring, piperidine or pyrrolidine ring; an unsubstituted or substituted phenyl ring wherein the substituents are selected from halo, C₁-C₄ alkyl or C₁-C₄ alkoxy; a 3-methyl-1,2,4-oxadiazol-5-yl group; a 2- or 3-thienyl group; a 2-, 3-, or 4-pyridyl group; a 4-pyrazolyl group; a 2-imidazole group optionally substituted at the N atom with a methyl group; a 2-thiazole group optionally substituted at the 4-position with a methyl; a ketone of structure C(O)R₁₉ wherein R₁₉ is C₁-C₃ alkyl, phenyl or 1-methylimidazol-2-yl; an ether CH₂0R₂₀ where R₂₀ is C₁-C₃ alkyl; a thioether - CH₂SR₂₀; a sulfone - CH₂SO₂CH₃; an amine - CH₂N(R₂ₒ)₂;an amine derivative -CH₂NHC(O)R₂₁ where R₂₁ is CH₃, NH₂ or NHCH₃; -CH₂NHS0₂NMe₂; or a carbamate - CH₂0C(O)NHMe;
R₅ and R₆ are independently of each other hydrogen or methyl; and
n is an integer 0, 1 or 2;
and salts thereof;
in racemic form, or the pure or substantially pure enantiomer thereof;
with the proviso that Y and Z are not both N, and with the further provisos that the following combination of substituents do not occur simultaneously:
(i) Y and Z are both carbon; R₁ or R₂ are hydrogen, halo, C₁-C₄ alkyl, C₁-C₄ alkoxy, CN, N0₂ or CF₃; R₃ is an unsubstituted phenyl; and R₄ is -C(O)OR_{16'} wherein R_{16'} is hydrogen, alkyl, alkenyl or alkynyl; or R₄ is -C(O)N(R₁₈,)(R_{19'}) wherein R_{18'} and R_{19'} are hydrogen, C₁-C₆ alkyl, phenyl or alkoxy or together with the N atom form a pyrrolidine, piperidine or morpholine ring; or R₄ is -CN or -C(S)-NH₂;
or (ii) Y and Z are both carbon; R₄ is C(O)OCH₃; R₁ and R₂ are both hydrogen, and R₃ is 4-hydroxyphenyl, unsubstituted phenyl or a 4-imidazole group.

2. Compounds of formula (I) as claimed in claim 1 wherein X, Y, Z, R₁, R₂, R₄, Rs, R₆ and n are as defined in claim 1, and R₃ is cyclohexyl or an optionally substituted phenyl ring wherein the substituents are selected from halo, CF₃, C₁-C₄ alkyl, C₁ -C₄ alkoxy; -SO₂R₁₂, NHC(O)R₁₂, HNSO₂R₁₂, SO₂NR₁₃R₁ in which R₁₂, R₁ and R₁ are as defined in claim 1; N0₂; or R₃ may be a pyridine ring, a morpholine ring, a pyrrolidine ring, a piperidino or an imidazole ring optionally substituted on nitrogen with C₁-C₄ alkyl, preferably compounds wherein X is O, and Y and Z are C.

3. Compounds of formula (I) as claimed in claim 2 wherein
R₁ and R₂ are independently C₁-C₆ alkyl, halo, CF₃, C₁-C₆ alkoxy or -SC₂NR_{1 3}R₁₄, wherein R_{1 3} and
R₁ are hydrogen or C₁-C₆ alkyl;
R₃ is cyclohexyl; an optionally substituted phenyl ring wherein the substituents are selected from halo, CF₃, C₁-C₄ alkyl and C₁-C₄ alkoxy; a 1-piperidinyl ring or pyridine ring;
R₄ is an optionally substituted phenyl ring wherein the substituents are selected from halo, C₁ -C₄ alkyl and C₁-C₄ alkoxy; a 3-methyl-1,2,4-oxadiaxol-5-yl group; a 2-thienyl group; a 2-, 3-, or 4-pyridyl group;
or a 1-methylimidazol-2-yl group;
and n is 1 or 2.

4. Compounds as claimed in Claim 2 wherein
R₁ is in the 5-position and is C₁ -Ca alkyl or halo,
R₂ is hydrogen,
R₃ is cyclohexyl,
R₄ is 2-pyridyl or 3-pyridyl,
R₅ is hydrogen,
R₆ is hydrogen,
X is 0 or S.
and n is 1.

5. A compound as claimed in claim 1 selected from
2-(2-Cyclohexyl-1-phenyl) ethylaminobenzoxazole;
2-{[2-(Cyclohexyl-1-(2-pyridyl)ethyl]amino}benzoxazole
L-2-{[2-cyclohexyl-1-(2-pyridyl)ethyl]amino)benzoxazole
2-{[2-Cyclohexyl-1-(2-pyridyl)ethyl]amino}-5-methylbenzoxazole
L-2-{(2-Cyclohexyl-1-phenyl)ethylamino}-5-isopropylbenzoxazole
2-{[2-Cyclohexyl-1-(2-pyridyl)ethyl]amino}-5-chlorobenzoxazole;and salts thereof.

6. Compounds of formula (X) 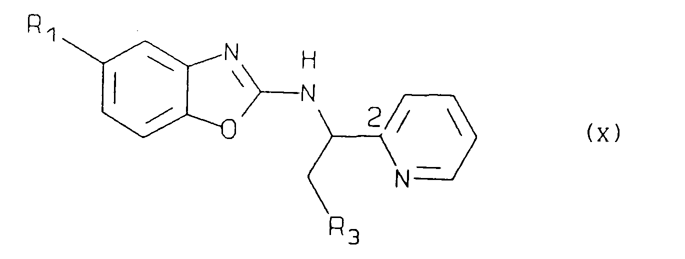
wherein
R₁ is isopropyl, methyl, chloro or methoxy and
R₃ is 4-fluorophenyl.

7. Compounds of formula (XI) 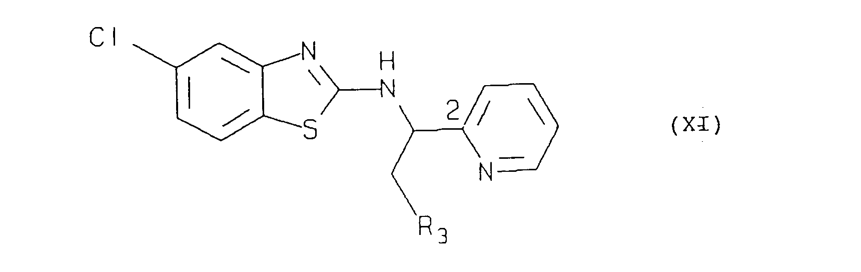
wherein
R₃ is 4-fluorophenyl or cyclohexyl.

8. A compound of formula (XII) 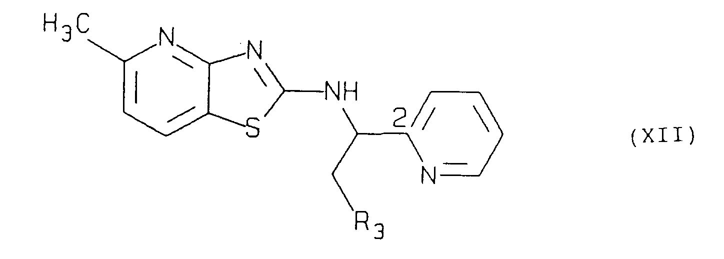
wherein
R₃ is cyclohexyl.

9. A compound of formula (XIII) 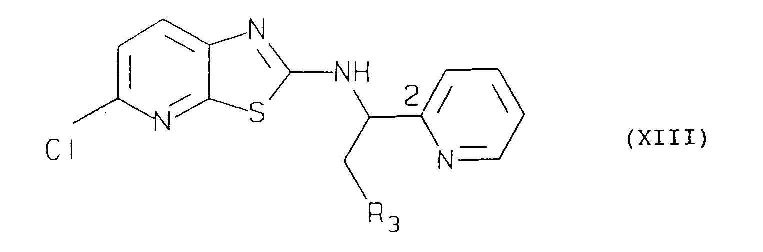
wherein
R₃ is cyclohexyl.

10. The compound as claimed in any one of claims 1 to 9, the L-enantiomer thereof.

11. Process for the preparation of a compound of general formula I as claimed in any one of claims 1 to 10, characterized in that
A) a compound 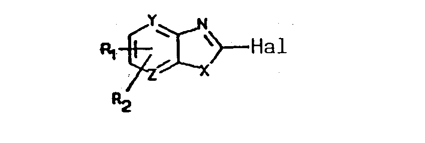
is reacted with an amine 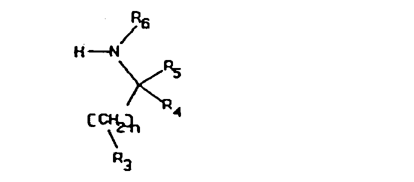
or reactive derivative thereof (wherein X, Y, Z, Ri, R₂, R₃, R₄, Rs, R₆ and n are as defined in any one of claims 1 to 10, in an inert solvent in the presence of a basic catalyst;
B) for the preparation of a compound I where X is S, Y and Z are C and R⁶ is H a compound 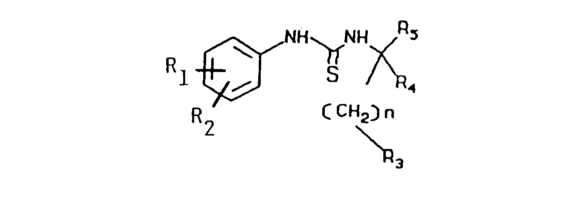
wherein Ri, R₂, R₃, R₄, R₅ and n are defined as in claim 1 is cyclized;
C) for the preparation of a compound I wherein X is S, Y is C and Z is N and Ri, R₂, R₃, R₄, Rs, R₆ and n are defined as in any one of claims 1 to 10,
a compound 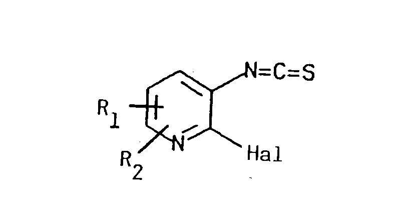
is reacted with an amine 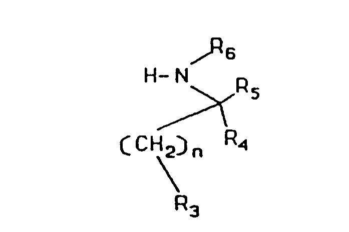
or reactive derivative thereof
wherein R₁, R₂, R₃, R₄, Rs, R₆ and n are as defined above and Hal is halogen;
D) for the preparation of a compound I wherein X is S, Y is N and Z is C and Ri, R₂, R₃, R₄, Rs, R₆ and n are defined as in claim 1 a compound 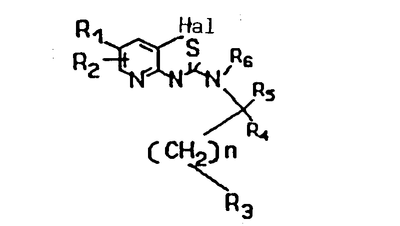
wherein R₁, R₂, P₃, R₄, Rs, R₆ and n are defined as above and Hal is halogen, is cyclized;
E) for the preparation of a compound I wherein R₄ is -CO₂R₁₆ and X, Y, Z, R₁, R₂, R₃, Rs, R₆, R₁₆ and n are as defined in claim 1
reaction of an acid 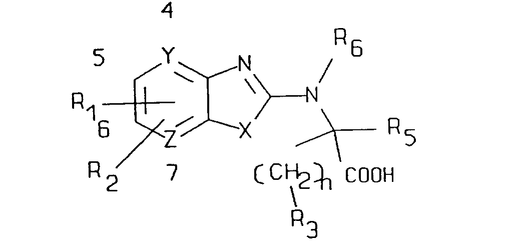
with an alcohol R₁₆OH or a reactive derivative thereof,
wherein X, Y, Z, R₁, R₂, R₃, Rₛ, R₆, R₁₆ and n are as defined above;
F) for the preparation of a compound I wherein R₄ is -C(O)NR₁₇R₁₈ and X, Y, Z, R₁, R₂, R₃, Rs, R₆, R₁₇, R₁₈ and n are as defined in claim 1 reaction of an acid 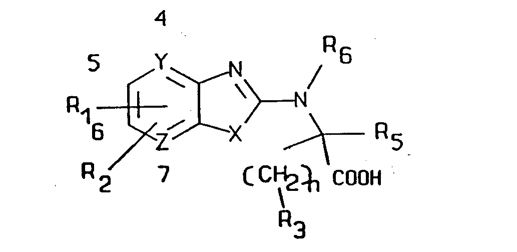
with an appropriately substituted amine NHR₁₇R₁₈, wherein R₁₇ and R₁₈ are defined as above;
G) for the preparation of a compound I wherein R₄ is -C(O)CH₃ and X, Y, Z, Ri, R₂, R₃, R₅, R₆, R₁₉ and n are defined as in claim 1
reaction of a compound 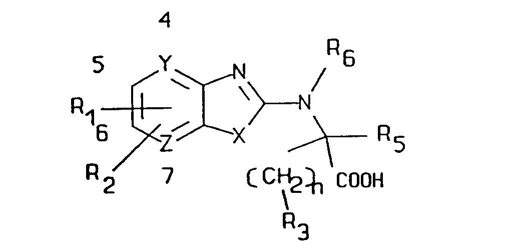
with lithium methyl;
H) for the preparation of a compound I wherein R₄ is 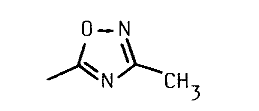
and X, Y, Z, R₁, R₂, R₃, Rₛ, R₆ and n is as defined in claim 1
reaction of a compound 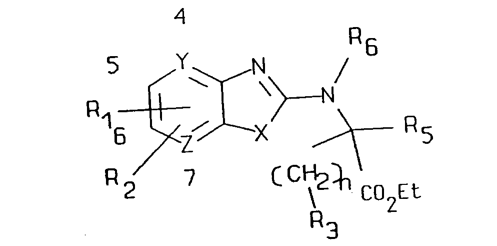
wherein X, Y, Z, R₁, R₂, R₃, Rₛ, R₆ and n are defined as above with acetamidoxime;
I) for the preparation of a compound I wherein R₄ is -CH₂OR₂₀ or -CH₂SR₂₀ and X, Y, Z, Ri, R₂, R₃, Rs, R₆, R₂₀ and n are defined as in claim 1
reaction of a compound 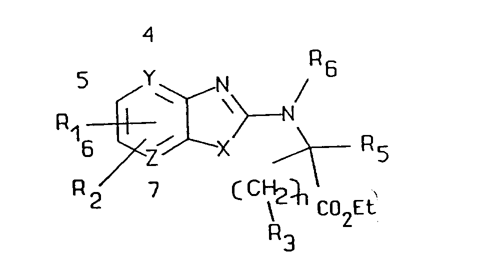
wherein X, Y, Z, R₁, R₂, R₃, Rₛ, R₆ and n are as defined above with NaOR₂o or NaSR₂o, respectively;
followed by the isolation of the racemate and/or the individual enantiomer and/or by salt formation.

12. Pharmaceutical compositions comprising as active ingredient at least one compound of formula (I) 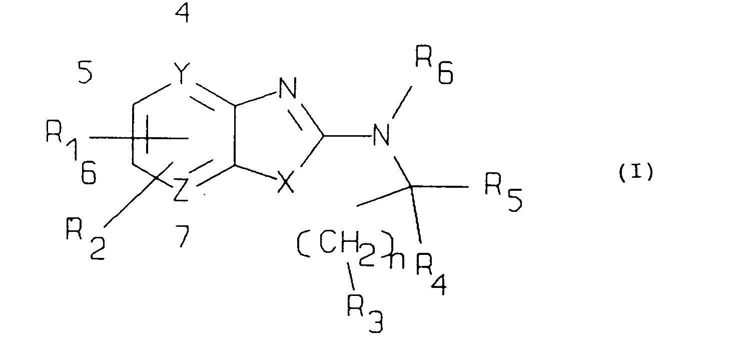
wherein
X is O or S;
Y is C or N;
Z is C or N;
with the proviso that Y and Z are not both N;
R₁ and R₂ are each, independently of one another, hydrogen; C₁-C₆ alkyl; halo; CF₃; nitrile; C₁-C₆ alkoxy; -CO₂R₇ wherein R₇ is hydrogen or C₁-C₆ alkyl; -C(O)NR₈R₉ wherein R₈ and R₉ are independently, hydrogen, C₁-C₃-
alkyl, methoxy or together with the N atom form a morpholine, pyrrolidine or piperidine ring; -N0₂; -NR₁₀R₁₁ wherein R₁ and R₁₁ are independently hydrogen or C₁-C₆ alkyl;-C(O)R₁ wherein R ₁₂ is C₁-C₆ alkyl;-SO₂R₁₂; -NHC(O)R₁₂;-NHSO₂R₁₂; or-SO₂NR₁₃R₁₄ wherein R₁₃ and R₁₄ are independently hydrogen or C₁-C₆ alkyl;
R₃ is methyl, cyclohexyl or an optionally substituted phenyl ring wherein the substituents are selected from halo, CF₃, C₁-C₄ alkyl and C₁-C₄ alkoxy; -SO₂R₁₂, NHC(O)R₁₂, -NHSO₂R₁₂, or - SO₂NR₁₃R₁₄ in which R₁₂, R₁₃ and R₁₄ are as defined above; N0₂; a 1-piperidinyl ring, a 2-, 3- or 4- pyridine ring, a morpholine ring, a thiomorpholine ring, a pyrrolidine ring, or an imidazole ring optionally substituted at the nitrogen atom with C₁-C₄ alkyl, a 2-thiazole ring or a 2-methyl-4-thiazole ring; a dialkylamine (C₁-C₄) or an alkyl ether (C₁-C₄);
R₄ is an ester -CO₂R₁ wherein R₁ is G₁-C₄ alkyl; or an amide of structure -C(O)NR₁₇ R ₁₈ wherein R₁₇ and R₁₈ are independently hydrogen, C₁-C₃ alkyl, methoxy or together with the N atom form a morpholine ring, or together with the N atom form a piperidine or pyrrolidine ring; an optionally substituted phenyl ring wherein the substituents are selected from halo, C₁-C₄ alkyl and C₁-C₄ alkoxy;
a 3-methyl-1,2,4-oxadiazol-5-yl group; a 2- or 3-thienyl group; a 2-, 3-, or 4-pyridyl group; a 4-pyrazolyl group; a 2-imidazole group optionally substituted on N with a methyl group; a 2-thiazole group optionally substituted on the 4-position with a methyl; a ketone C(O)R₁₉ wherein R₁₉ is C₁-C₃ alkyl, phenyl or 1-methylimidazol-2-yl; an ether -CH₂0R₂₀ where R₂₀ is C₁-C₃ alkyl; a thioether, -CH₂SR₂₀; a sulfone, -CH₂SO₂CH₃; an amine, -CH₂N(R₂₀)₂; an amine derivative, -CH₂NHC(O)R₂₁ where R₂₁ isCH₃, NH₂or NHCH₃;
-CH₂NHS0₂NMe₂ ; or a carbamate, -CH₂OC(O)NHMe;
R₅ and R₆ are independently of each other hydrogen or methyl; and
n is an integer 0, 1 or 2;
in racemic form, or the pure or substantially pure enantiomer thereof, or a physiologically acceptable acid addition salt thereof in association with one or more pharmaceutically acceptable carriers, diluents or excipients.

13. Pharmaceutical compositions as claimed in claim 12 wherein the compounds of formula (I) are as defined in any one of claims 1 to 10.

14. The use of a compound of formula (I) as defined in any one of claims 1 to 10, 12 or 13 or a physiologically acceptable salt thereof for the preparation of a medicament for use in the prevention or treatment of inflammatory conditions and conditions or diseases in which arachidonic acid metabolism has been implicated.

15. The use as claimed in claim 14 for the preparation of a medicament for the inhibition of the biosynthesis of leukotrienes.
